# EUROPÄISCHE PATENTANMELDUNG

(11) **EP 1 219 603 A2**
(43) Veröffentlichungstag der Anmeldung: **03.07.2002**
(21) Anmeldenummer: 01130523.2
(22) Anmeldetag: 21.12.2001
(51) Int. Cl.: C07D 209/20

(54) **Tryptophan-Analoga in Proteinen, Peptiden und peptidischen Leitstrukturen**

(30) Priorität: 21.12.2000 DE 10063992
(71) Anmelder: Max-Planck-Gesellschaft zur Förderung der Wissenschaften e.V., 80539 München (DE); proteros biostructures GbmH, 82152 Martinsried (DE)
(72) Erfinder: Huber, Robert, Prof. Dr., 82110 Germering (DE); Budisa, Nediljko, Dr., 85622 Feldkirchen (DE); Moroder, Luis, Prof., 82152 Martinsried (DE); Alefelder, Stefan, Dr., 80805 München (DE); Bae, Jae Hyun, 82061 Neuried (DE); Kaiser, Jens, Dr., 81369 München (DE); Freye-Minks, Caroline, Dr., Uni of Virginia, Charlottesville, VA 22809 (US); Neuefeind, Torsten, Dr., 82131 Gauting (DE)
(74) Vertreter: Maiwald Patentanwalts GmbH

(57) **Zusammenfassung**

Die Erfindung betrifft Proteine, Peptide und peptidische Leitstrukturen, die aufgrund des Einbaus von Tryptophan-Aminosäureanaloga, insbesondere von Chalkogen- und/oder Halogen-Analoga, neue kristallographische, spektrale und/oder pharmakologische Eigenschaften aufweisen. Ferner betrifft die Erfindung ein Verfahren zur Markierung von Proteinen, Peptiden und peptidischen Leitstrukturen, bei dem diese durch den Einbau von Aminosäure-Analoga mit neuen kristallographischen und/oder spektralen Eigenschaften versehen werden. Des Weiteren betrifft die Erfindung ein Verfahren zur Bestimmung der dreidimensionalen Struktur von Proteinen, Peptiden oder peptidischen Leitstrukturen mittels Kristallstrukturanalyse. Schließlich betrifft die Erfindung pharmazeutische Zusammensetzungen, die als Wirkstoff mindestens ein Tryptophan-Analogon umfassen, das in einen Träger, beispielsweise ein Protein, ein Peptid oder eine peptidische Leitstruktur, eingebaut ist.

## Beschreibung

Die Erfindung betrifft Proteine, Peptide und peptidische Leitstrukturen, die aufgrund des Einbaus von Tryptophan-Aminosäureanaloga, insbesondere von Chalkogen- und/oder Halogen-Analoga, neue kristallographische, spektrale und/oder pharmakologische Eigenschaften aufweisen. Ferner betrifft die Erfindung ein Verfahren zur Markierung von Proteinen, Peptiden und peptidischen Leitstrukturen, bei dem diese durch den Einbau von Aminosäure-Analoga mit neuen kristallographischen und/oder spektralen Eigenschaften versehen werden. Des Weiteren betrifft die Erfindung ein Verfahren zur Bestimmung der dreidimensionalen Struktur von Proteinen, Peptiden oder peptidischen Leitstrukturen mittels Kristallstrukturanalyse. Schließlich betrifft die Erfindung pharmazeutische Zusammensetzungen, die als Wirkstoff mindestens ein Tryptophan-Analogon umfassen, das in einen Träger, beispielsweise ein Protein, ein Peptid oder eine peptidische Leitstruktur, eingebaut ist.

Grenzen für die biologische Forschung bestehen heutzutage hauptsächlich darin, aus den riesigen Mengen an vorhandenen Gensequenzdaten Kenntnisse über die Struktur und Aktivität der Genprodukte auf molekularer Ebene abzuleiten. In diesem Zusammenhang ist die Proteom-Analyse ("proteomics", proteins expressed by genomes) zu einem unverzichtbaren Partner der Genomanalyse ("genomics") geworden. Unter dem Proteom versteht man die Summe der zu einem gegebenen Zeitpunkt und unter bestimmten Umgebungsbedingungen exprimierten Proteine einer Zelle einschließlich aller posttranslationalen Veränderungen dieser Proteine. Damit ist das Proteom im Gegensatz zum statischen Genom eine dynamische Größe.

Zur Analyse der Genprodukte aus den Gensequenzen eines Organismus kann beispielsweise zunächst jedes unbekannte Gen in ein geeignetes Plasmid eingefügt werden und unter Kontrolle eines geeigneten Promotors beispielsweise in *E. coli* heterolog exprimiert werden. Da zu einem gegebenen Zeitpunkt unter herkömmlichen Expressionsbedingungen in einer Zelle viele tausend Proteine exprimiert werden, und diese in sehr unterschiedlichen Mengen, tritt das Problem auf, das angestrebte Proteinprodukt zu identifizieren und von den übrigen z. B. in *E. coli* exprimierten Proteinen zu isolieren. Prinzipiell lassen sich die Proteine durch chromatographische und elektrophoretische Verfahren auftrennen, wie beispielsweise durch zweidimensionale Gelelektrofokussierung (eine Dimension isoelektrische Fokussierung (IEF), eine Dimension Polyacrylamid-Gelelektrophorese (PAGE)). Die Identifizierung der gewünschten Proteinprodukte ist unter anderem durch Aktivitätsassays oder spezifische Antikörper ("Western Blot") sowie durch weitere Proteinanalytik, wie beispielsweise Peptidspaltung und Sequenzierung durch Massenspektrometrie, möglich. Dabei wird die Analyse durch jede Art von Informationen erleichtert, die die Eigenschaften des Zielproteins betreffen, wie beispielsweise die Gegenwart von unterschiedlich gefärbten Cofaktoren oder eine spezifische enzymatische Aktivität. Nachteilig an den bisher bekannten Verfahren ist insbesondere, dass häufig die Verwendung von hochspezifischen Antikörpern notwendig ist.

Zusätzlich macht es die Dynamik des Proteoms schwer, eine ausreichende Vergleichbarkeit der experimentellen Bedingungen und daraus resultierende identische Proteinmuster beispielsweise in der zweidimensionalen Gelelektrophorese zu erreichen. Deshalb ist es bislang bei der Proteom-Analyse noch nicht möglich, das gesamte Proteom einer Zelle oder eines Gewebes quantitativ zu beschreiben. Vielmehr geht es bislang eher darum, die unterschiedliche Proteinexpression in miteinander in Beziehung stehenden, nicht zu weit voneinander entfernten Zuständen zu analysieren. Beispiele sind die Veränderung der Proteinexpression einer Zelle bei veränderten Umgebungsbedingungen, beispielsweise Temperatur- oder mechanischer Belastung, zusätzlicher Anwesenheit eines Wirkstoffs oder Fortschreiten der zellulären Differenzierung. Eine solche differentielle Analyse kann aber nur erfolgreich sein, wenn die ausgewählten zellulären Zustände sicher definiert sind.

Die Proteom-Analyse ist bereits zu einem der wichtigsten Forschungszweige der Pharmaentwicklung geworden. Zum einen eignet sie sich zur Auffindung von neuen Zielmolekülen ("Targets") für Arzneimittelkandidaten und Agrochemikalien, zum anderen insbesondere für die Targetbewertung. So lässt sich durch die Proteom-Analyse das gesamte Spektrum der Wirkungen einer Substanz auf eine Zelle oder ein Gewebe untersuchen, also die Einflüsse auf Zellstoffwechsel, Zellwandeigenschaften, DNA- und Proteinsynthese usw.

Ebenso ermöglicht die Proteom-Analyse pharmakologische und toxikologische Untersuchungen. Mit den substanzbezogenen Expressionsmustern lassen sich "Marker für die Wirksamkeit" einer Substanz definieren, die dann wiederum für die Optimierung von peptidischen Leitstrukturen für Arzneimittelkandidaten, beispielsweise im Rahmen des "metabolic engineering", verwendet werden können.

Unter peptidischen Leitstrukturen sind im Rahmen der vorliegenden Erfindung biologisch aktive Wirkstoffe zu verstehen, die unter anderem oder ausschließlich aus Aminosäuren und/oder Aminosäureanaloga aufgebaut sind und als strukturelle Basis für die Entwicklung von pharmakologisch verwendbaren Wirkstoffen beispielsweise mit Hilfe von High-Throughput-Screening und kombinatorischer Chemie dienen.

Somit ist es ein essentielles Ziel der Proteom-Forschung, die gegenwärtig erhältlichen Strategien zur schnellen und verlässlichen Analyse der gesamten Proteinprodukte aus den Gensequenzen der Vielfalt von Organismen zu verbessern oder neue Strategien zu entwickeln. Eine spezifische biosynthetische Einführung von nicht-invasiven Markern, die die Identifizierung von Zielproteinen in Reinigungs- und Charakterisierungsverfahren erleichtern, könnte deshalb insbesondere zur Analyse der Struktur-/Funktionsbeziehungen sehr vorteilhaft sein.

Im Zusammenhang mit der vorliegenden Erfindung werden die Begriffe Zielprotein bzw. Targetprotein für Proteine verwendet, deren Identifizierung und Charakterisierung, insbesondere in struktureller Hinsicht, erwünscht ist.

In diesem Zusammenhang wäre es von großer Bedeutung, ein Aminosäureanalogon zu finden, das intrinsisch neue Eigenschaften wie beispielsweise neue spektrale Fenster mit sich bringt, die sich von jenen der durch den genetischen Code vorgeschriebenen zwanzig kanonischen Aminosäuren unterscheiden. Eine derartige Erweiterung des Aminosäurerepertoires des genetischen Codes ist ein attraktives und herausforderndes Ziel und wird durch verschiedene in vitro- und in vivo-Verfahren erreicht.

Erste praktische Anwendungen des Protein-Engineerings durch Einbau von Aminosäuren mit neuen Eigenschaften waren die Markierung von Proteinen mit ³⁵S-Methionin für radioaktive Assays (D. Plesse, Z. f. Naturforschung, C, Biosciences 291, 42-47 (1974)) und später die Markierung von aromatischen Aminosäuren mit ¹⁹F für die NMR-Strukturbestimmung (E. Pratt et al., Biochemistry 13, 3035-3040 (1975)). Anfang der 90er Jahre stellte der isostere Ersatz von Methionin durch Selenomethionin und Telluromethionin (W. Hendrickson et al., Science, 254, 51-58 (1991); J. Boles et al., Nature Struct. Biol. 30, 283-284 (1994); N. Budisa et al., Eur. J. Biochemistry 230, 788-796 (1995)) ein nützliches Werkzeug für die routinemäßige kristallographische Strukturbestimmung von Proteinen dar.

Da die gegenwärtig erhältlichen in vitro-Verfahren unter schwerwiegenden Nachteilen, wie geringen Ausbeuten im Submilligrammbereich, sowie teuren und zeitaufwendigen Protokollen leiden, wurde in der deutschen Patentanmeldung DE 197 24 670 ein effizientes Verfahren für den Einbau von Aminosäureanaloga in Proteine unter starkem Selektionsdruck (selective pressure incorporation, SPI) bereitgestellt. Diverse nicht-kanonische Aminosäuren, die ihren entsprechenden kanonischen Aminosäuren in Form, Größe und chemischen Eigenschaften gleichen, können durch die SPI-Methode in rekombinante Proteine eingeführt werden, wobei die Ausbeuten ähnlich zu denen der Wildtyp-Formen sind. Auf diese Weise ist eine nicht-invasive Markierung von Proteinen möglich. Der hohe Grad an Inkorporation dieser Aminosäureanaloga in Aminosäure-spezifischer Weise wird durch einen hohen Selektionsdruck erreicht, indem genetisch veränderte, aber intakte Wirtsexpressionszellen in Verbindung mit einer Manipulierung der Aminosäurezuführung in einem definierten Medium verwendet werden. Dieses Verfahren führt zu einer neuen Form des Protein-Engineerings; es bietet die Möglichkeit der Einführung spezifischer chemischer, sterischer oder biophysikalischer Eigenschaften in Proteine, welche nicht durch den Standardsatz an kanonischen Aminosäuren verändert werden können. Ein derartiges Protein-Engineering wird nicht durch Manipulation des Codes auf DNA-Ebene (z.B. Oligonukleotid-gerichtete Mutagenese), sondern durch neue Zuordnung des Codes auf der Ebene der Proteintranslation unter effizientem Selektionsdruck durchgeführt.

Bei der SPI-Methode tritt nach Induktion des Targetgens über einen geeigneten Promotor normalerweise keine bzw. fast keine Produktion der zelleigenen Proteine mehr auf, da im Idealfall die gesamte Proteinsynthesemaschinerie das Targetprotein herstellt. Das derart hergestellte Targetprotein muß dann aus den zelleigenen Proteinen, die während der Wachstumsphase hergestellt worden sind, identifiziert werden.

Für das Verständnis der Funktion eines Proteins oder eines Peptids ist häufig die Kenntnis seiner dreidimensionalen Struktur von großer Bedeutung. Dabei ist es für die Aufklärung von Proteinstrukturen durch Röntgenstrukturanalyse und/oder NMR-Spektroskopie wesentlich, ein Modell zu generieren, das der wirklichen Situation in der Zelle nahe kommt.

Bei der Kristallstrukturanalyse wird zur Berechnung der Fourier-Synthese nicht nur die Amplitude der Streuwellen bzw. Strukturfaktoren, sondern auch deren Phase benötigt. Die Phase ist jedoch nicht über ein Experiment messbar, sie muß erst durch mehr oder weniger zeitaufwendige Verfahren bestimmt werden. Zur Lösung dieses sog. Phasenproblems der Kristallstrukturanalyse wird überwiegend die Methode der Schweratom-Derivatisierung eingesetzt. Hierbei werden beispielsweise die Proteinkristalle mit Schweratomverbindungen, wie Kaliumtetrachloroplatinat, getränkt und die Strukturfaktoramplituden verglichen. Falls die Schweratome an wenige definierte Bindungsstellen am Makromolekül binden, ist es möglich, durch die Patterson-Synthese ihre Positionen zu bestimmen, und dann, nach Interpretation mehrerer dieser Schweratomderivate, in einem zweiten Schritt die Phasen zu berechnen.

Die Suche nach geeigneten Schweratomverbindungen kann allerdings sehr zeitaufwendig sein. Veränderungen der Konstitution, wie sie durch das Einbringen von Schweratomen oder sogar Metallclustern vorgenommen werden, können ferner die native Struktur beeinflussen. Der *in vivo*-Einbau einer Schweratom-haltigen Aminosäure, die zu der nativen Aminosäure, die sie ersetzt, isoster ist, ist deshalb eine attraktive Alternative zu herkömmlichen Methoden der Phasenbestimmung bei der Kristallstrukturanalyse.

In den letzten Jahren wurde mit der MAD-Methode (MAD, multi-wavelength-anomalous-Dispersion) eine wertvolle Alternative zur Methode der multiplen Schweratomderivate entwickelt. Diese Methode beruht auf der anomalen Streuung der Atome. Für ein gegebenes Element ist der Beitrag der anomalen Streuung zur gemessenen Strukturfaktoramplitude von der verwendeten Wellenlänge abhängig. Nimmt man denselben Kristall bei verschiedenen Wellenlängen, so können die Unterschiede in den Strukturfaktoramplituden in Analogie zu verschiedenen Schweratomderivaten behandelt werden und zur Phasenbestimmung genutzt werden. Da die Änderungen sehr klein sind, sollten die Messungen wegen der benötigten Genauigkeit an Synchrotonen durchgeführt werden. Bei den am Synchroton einstellbaren Wellenlängen bieten sich beispielsweise Eisen und Selen als anomale Streuer an. Eisen kommt in vielen biologischen Strukturen als fester Bestandteil vor, beispielsweise in der Hämgruppe der Globine. Selen kann durch Substitution von Methionin durch Selenomethionin während der Proteinsynthese in Methionin-auxotophe *E.coli*-Stämmen eingeführt werden. Die MAD-Methode ist sehr elegant, da nur ein einziger Kristall zur Strukturanalyse benötigt wird und auch die zeitaufwendige Suche nach Schweratomderivaten entfällt.

Der Effekt des Einbaus von Selen in Proteine wurde bereits von Cowie und Cohen untersucht (Biochem. Biophys. Acta 1957, 26, 252 - 261). Die Optimierung der Fermentationsbedingungen sowie die kommerzielle Erhältlichkeit eines stabilen Methionin-auxotrophen Baktierienstamms (Budisa et al., Eur. J. Biochem. 1995, 230, 788 - 796) konnten bereits zur Lösung von Proteinstrukturen mittels Einbau von Selenomethionin führen. Proteine, die Selenomethionin enthalten, zeigen ähnliche Eigenschaften, können allerdings eine geringfügig verringerte Stabilität aufweisen. Basis für den Einbau dieser Aminosäurederivate ist die strukturelle Ähnlichkeit zwischen dem Analogon Selenomethionin und der nativen Aminosäure Methionin.

Nachteilig bei der Verwendung von Selenomethionin zur Röntgenstrukturanalyse basierend auf der MAD-Methode ist allerdings das häufige Vorkommen der Aminosäure Methionin in Proteinsequenzen (durchschnittlich 5 bis 7 Methionin-Reste pro 300 Aminosäuren), da die Gegenwart von zu vielen Resonanzzentren die Auswertung der Datensätze erschwert.

Deswegen wäre es für die Kristallstrukturanalyse wünschenswert, Schweratom- und/oder anomale Streuer enthaltende Aminosäure-Analoga in Proteine, Peptide oder peptidische Leitstrukturen einzubauen, die in sehr geringer Anzahl, idealerweise nur einmal pro Sequenz vorkommen. Eine wesentliche Aufgabe der vorliegenden Erfindung ist es somit, Proteine, Peptide oder peptidische Leitstrukturen bereitzustellen, in die für die Kristallstrukturanalyse geeignete Schweratom- und/oder anomale Streuer enthaltende Aminosäureanaloga inkorporiert sind.

Neben der Kristallstrukturanalyse profitiert auch die Kernresonanzspektroskopie (NMR, nuclear magnetic resonance) von den Eigenschaften nicht-kanonischer Aminosäuren. Die Strukturaufklärung von Proteinen mittels NMR kann durch den Einbau eines Analogons, das einen NMR-aktiven Kern trägt, der sich von den üblicherweise in der Protein-NMR-Spektroskopie detektierten Wasserstoff-, Kohlenstoff- und Stickstoffkernen unterscheidet, erleichtert werden. Deswegen ist es eine weitere Aufgabe der vorliegenden Erfindung, Proteine, Peptide und peptidische Leitstrukturen bereitzustellen, die Aminosäure-Analoga mit NMR-aktiven Kernen umfassen, die sich von ¹H, ¹³C und ¹⁵N unterscheiden.

Seit kurzem erlaubt ein erweitertes Aminosäurerepertoire *in vivo* die UV- und Fluoreszenzspektroskopischen Eigenschaften eines Targetproteins zu manipulieren (M. Ring et al., Biochem. Biophys. Res. Com. 131, 675-680 (1985); S. Beiboer et al., J. Mol. Biol. 260, 628-643 (1996); C. Minks et al., Biochemistry 38, 10649-10659 (1999); J.B. Ross et al., Method. Enzymol. 278, 151-190 (1997)).

So wurde beispielsweise bei menschlichem rekombinantem Annexin V (320 Aminosäuren) ein einzelner Tryptophan-Rest (Trp 187) mit Hilfe des SPI-Verfahrens durch drei unterschiedliche isostere Analoga ersetzt, bei denen die Wasserstoffatome an den Positionen 4, 5 bzw. 6 des Indolrings von Trp durch Fluoratome ersetzt waren (Minks et al., Biochemistry 1999, 38, 10649 - 10659).

Im Hinblick auf eine schnelle und zuverlässige Analyse von derart markierten Proteinsequenzen bzw. Proteinstrukturen ist es eine weitere Aufgabe der vorliegenden Erfindung, ein einfaches, zuverlässiges, kostengünstiges und leicht reproduzierbares Verfahren bereitzustellen, mit dem die Analyse und das Tracing von Proteinen, z.B. bei der Proteom-Analyse, erleichtert und beschleunigt werden können. Zu diesem Zweck bieten sich UV- und Fluoreszenzspektroskopie an.

Die hohe Empfindlichkeit der Fluoreszenzspektroskopie sowie die Geschwindigkeit des zugrundeliegenden physikalischen Prozesses erlauben die Erforschung schneller biologischer Vorgänge und die Beobachtung der Umgebung von Biomolekülen während einer Enzymreaktion. Die Fluoreszenz biologischer Systeme beruht auf der Anregung aromatischer, gegebenenfalls heterozyklischer Ringsysteme aus dem Grundzustand S₀ in angeregte höhere Zustände. Tryptophan spielt als interner Fluorophor in Proteinen eine Schlüsselrolle bei der Untersuchung von entweder dynamischen Vorgängen (time-resolved fluorescence) oder bei der Charakterisierung von Tertiärkonformationen von Proteinen (steady-state fluorescence). In nativen Proteinen ist der Beitrag von Phenylalaninen und Tyrosinen aufgrund ihrer geringen Quantenausbeuten in Gegenwart von Tryptophan vernachlässigbar. Während Tryptophan einen geeigneten Marker für die Untersuchung von Proteinstrukturen und des dynamischen Verhaltens von Proteinen in Lösung darstellt, wird die Beobachtung von Protein-Protein-Komplexen oder Protein-Nukleinsäure-Komplexen durch die Überlagerung der Emissionsmaxima der beteiligten Fluorophore erschwert.

Somit ist es eine Aufgabe der vorliegenden Erfindung, nicht-kanonische Derivate von Tryptophan bereitzustellen, die ein differenziertes UV- und/oder Fluoreszenzverhalten aufweisen und diese Eigenschaften auf das Protein, das Peptid oder die peptidische Leitstruktur übertragen, in das bzw. die sie eingebaut werden.

In den letzten Jahren sind große Anstrengungen aufgewendet worden, um neue und wirksame Methoden zur Verabreichung und insbesondere zur zielgerichteten gewebespezifischen Verabreichung von Arzneimitteln zu entwickeln. Arzneimittel unterscheiden sich jedoch stark hinsichtlich ihrer Größe, ihrer chemischen Natur und ihrer pharmakologischen Funktionen. Eine generelle Anwendbarkeit der bislang bekannten Methoden ist nicht möglich, da jede der im Folgenden genannten Voraussetzungen erfüllt werden muß:
1. Eindringen des Arzneimittels in den Körper,
2. Aufrechterhalten der biologischen Aktivität des Arzneimittels,
3. Transport zu seinem Wirkort, und
4. Abgabe des Arzneimittels am Wirkort in therapeutisch wirksamer Form.

Aminosäurederivate bzw. Aminosäure-Analoga spielen im menschlichen Organismus eine wichtige Rolle als Botenstoffe oder Intermediate im Metabolismus. Beispiele für körperfremde Substanzen, die sich bei der medizinischen Therapie als bedeutungsvoll erwiesen haben, sind Thioprolin, das eine wichtige Rolle als Thiol-Antioxidationsmittel, Radikalfänger und Anti-Tumormittel spielt, sowie Thienopyrrol, das ein bioisosteres Analogon des Halluzinogens und Serotonin-Agonisten N,N-Dimethyltryptamin ist (J.B. Blair et al., J. Med. Chem. 1999, 42, 1106-1111).

Pharmakologisch wirksame Aminosäure-Analoga sind bisher in freier Form auf verschiedene Art und Weise, zum Beispiel oral oder parenteral verabreicht worden. Dabei wurden jedoch oftmals unerwünschte starke Nebenwirkungen beobachtet.

Darüber hinaus wurden Aminosäure-Analoga durch chemische Synthese in Peptid-Analoga eingebaut, die selbst pharmakologische Eigenschaften, zum Beispiel als Enzyminhibitoren, besitzen (vergleiche zum Beispiel Baldwin et al., Structure 1995, 3, 581 - 590).

Neben seiner Rolle als Grundbaustein in der Ribosomen-vermittelten Proteinsynthese spielt Tryptophan eine wesentliche Rolle im Metabolismus von lebenden Zellen, da es in die Biosynthese von Hormonen wie Serotonin oder Melatonin in Tieren sowie von Indolalkaloiden in Pflanzen involviert ist (Philips et al., Bioorg. & Med. Chem. Lett. 1995, 5, 1133 - 1134). Deshalb besteht ein großes Interesse, nicht-kanonische Aminosäureanaloga von Tryptophan als potentielle Antagonisten, Wirkstoffe oder Antibiotika einzusetzen. Die Inkorporation dieser nicht-kanonischen Aminosäureanaloga von Tryptophan in Proteine ist ein vielsprechender Ansatz für die Herstellung von therapeutischen Mitteln (Budisa et al., Proc. Natl. Acad. Sci. USA 1998, 95, 455 - 459; Minks et al., Tetrahedron 2000, 56, 9431 - 9442).

Somit besteht eine weitere Aufgabe der vorliegenden Erfindung darin, neue pharmakologische Zusammensetzungen bereitzustellen, die als Wirkstoff pharmakologisch wirksame Aminosäure-Analoga umfassen.

Zur Lösung dieser und weiterer Aufgaben dienen die Merkmale der unabhängigen Schutzansprüche.

Vorteilhafte Ausgestaltungen sind in den jeweiligen Unteransprüchen definiert.

Erfindungsgemäß wird ein Protein, ein Peptid oder eine peptidische Leitstruktur bereitgestellt, das bzw. die mindestens ein Chalkogen-enthaltendes Aminosäure-Analogan von Tryptophan und/oder mindestens ein Halogen-enthaltendes Aminosäure-Analogan von Tryptophan umfasst, wobei das Chalkogen aus der Gruppe, bestehend aus Schwefel, Selen und Tellur, ausgewählt ist, und das Halogen aus der Gruppe, bestehend aus Brom und Iod, ausgewählt ist.

Unter einem Aminosäure-Analogon im Rahmen der vorliegenden Erfindung wird eine beliebige nicht-kanonische, mit einer kanonischen, genetisch kodierten Aminosäure in Bezug auf Struktur, d.h. Form und Größe, und chemischen Eigenschaften verwandte Verbindung verstanden. Als nicht-kanonische Aminosäuren werden solche bezeichnet, die nicht von den 20 kanonischen durch Nukleinsäuren kodierten α-Aminosäuren umfasst sind.

Unter Proteinen werden im Rahmen der vorliegenden Erfindung Biopolymere verstanden, die mindestens 70, vorzugsweise mindestens 80 und besonders bevorzugt mindestens 100 Aminosäure- bzw. Aminosäureanalogon-Reste umfassen. Proteine im Rahmen der vorliegenden Erfindung weisen vorzugsweise Molmassen von mindestens 7 kDa, vorzugsweise 8 kDa und besonders bevorzugt 10 kDa auf.

Vorzugsweise ist die Aromatizität in dem dem Indolring von Tryptophan entsprechenden Ringsystem des Aminosäure-Analogons erhalten.

Falls das Protein, Peptid oder die peptidische Leitstruktur ein Chalkogen-enthaltendes Aminosäure-Analogon von Tryptophan umfasst, ist es bevorzugt, dass das dem Indolring von Tryptophan entsprechende Ringsystem zwei kondensierte Fünfringe umfasst, wobei sich eine NH-Gruppe in dem einen Fünfring befindet und das Chalkogen in den anderen Fünfring eingebaut ist.

Bei einer weiteren bevorzugten Ausführungsform der vorliegenden Erfindung ist das Chalkogen Selen. Besonders bevorzugt wird das Aminosäure-Analogon von Tryptophan aus der Gruppe, bestehend aus β-Selenolo[3,2-b]pyrrolyl-L-alanin (im Folgenden auch als [3,2]Sep bezeichnet) und β-Selenolo[2,3-b]pyrrolyl-L-alanin ([2,3]Sep) ausgewählt.

Bei einer Ausführungsform der vorliegenden Erfindung ist das dem Indolring von Tryptophan entsprechende Ringsystem mit Halogenen substituiert.

Ebenfalls kann es bei der vorliegenden Erfindung bevorzugt sein, dass das Aminosäure-Analogon von Tryptophan aus der Gruppe, bestehend aus β-Thieno[3,2-b]pyrrolyl-L-alanin ([3,2]Tpa), β-Thieno[2,3-b]pyrrolyl-L-alanin ([2,3]Tpa), β-Tellurolo[3,2-b]pyrrolyl-L-alanin ([3,2]Tep) und β-Tellurolo[2,3-b]pyrrolyl-L-alanin ([2,3]Tep), 4-Bromo-Tryptophan, 5-Bromo-Tryptophan, 6-Bromo-Tryptophan, 4-Iodo-Tryptophan, 5-Iodo-Tryptophan und 6-Iodo-Tryptophan, ausgewählt wird.

Bei einer weiteren bevorzugten Ausführungsform ersetzt das Aminosäure-Analogon von Tryptophan eine kanonische Aminosäure in einem nativen Protein, einem nativen Peptid oder einer nativen peptidischen Leitstruktur. Besonders bevorzugt sind alle Tryptophan-Reste der Sequenz eines nativen Proteins, eines nativen Peptids oder einer nativen peptidischen Leitstruktur durch Aminosäure-Analoga von Tryptophan ersetzt.

Bei einer weiteren alternativen Ausführungsform der vorliegenden Erfindung ist das Chalkogen ⁷⁷Se.

Bei einem weiteren Aspekt der vorliegenden Erfindung wird ein Verfahren zur Markierung von Proteinen, Peptiden oder peptidischen Leitstrukturen für kristallographische und/oder spektroskopische Untersuchungen bereitgestellt, bei dem mindestens ein Chalkogen-enthaltendes Aminosäure-Analogon von Tryptophan und/oder mindestens ein Halogen-enthaltendes Aminosäure-Analogon von Tryptophan in ein Protein, ein Peptid oder eine peptidische Leitstruktur eingebaut wird, wobei das Chalkogen aus der Gruppe, bestehend aus Schwefel, Selen und Tellur, ausgewählt wird, und das Halogen aus der Gruppe, bestehend aus Brom und Iod, ausgewählt wird.

Die Herstellung des Proteins, des Peptids bzw. der peptidischen Leitstruktur kann rekombinant, synthetisch, semi-synthetisch oder in einem *in-vitro-*System erfolgen. Bevorzugt erfolgt der Einbau des Aminosäure-Analogons von Tryptophan in das Protein *in vivo* in einer Wirtszelle.

Ein besonders bevorzugtes erfindungsgemäßes Verfahren zum Einbau des Aminosäure-Analogons von Tryptophan in das Protein umfasst die folgenden Schritte:
a) Bereitstellen einer Wirtszelle mit einer für das Protein kodierenden Nukleinsäuresequenz in operativer Verknüpfung mit einer regulierbaren Expressionskontrollsequenz,
b) Kultivieren der Zelle unter Bedingungen, bei denen die Transkription der für das Peptid oder Protein kodierenden Nukleinsäuresequenz weitgehend abgeschaltet ist,
c) Zugeben des Aminosäure-Analogons von Tryptophan, und
d) Induzieren der Expression des Peptids oder Proteins.

Besonders bevorzugt wird als Wirtszelle eine prokaryontische Wirtszelle verwendet.

Ferner ist es bevorzugt, dass eine Wirtszelle verwendet wird, die für eine durch das Aminosäure-Analogon von Tryptophan zu ersetzende kanonische Aminosäure auxotroph ist, und bei Schritt a) des vorstehend genannten Verfahrens die zu ersetzende kanonische Aminosäure dem Medium zugesetzt wird.

Ein weiterer Gegenstand der vorliegenden Erfindung ist ein Verfahren zur Bestimmung der dreidimensionalen Struktur von Proteinen, Peptiden oder peptidischen Leitstrukturen mittels Kristallstrukturanalyse, das die folgenden Schritte umfasst:
a) Herstellung eines wie vorstehend beschriebenen erfindungsgemäßen Proteins, Peptids bzw. einer peptidischen Leitstruktur durch Substitution mindestens eines Tryptophanrests des nativen Proteins, des nativen Peptids bzw. der nativen peptidischen Leitstruktur durch ein Aminosäure-Analogon von Tryptophan;
b) Kristallisierung des in Schritt a) hergestellten Proteins, Peptids bzw. der peptidischen Leitstruktur;
c) Kristallstrukturanalyse des Kristalls des Proteins, Peptids bzw. der peptidischen Leitstruktur aus Schritt b).

Falls zur Bestimmung der Phasen der Streuwellen (Strukturfaktoren) in Schritt c) des vorstehend genannten Verfahrens die MAD-Methode eingesetzt wird, wird das Aminosäure-Analogon von Tryptophan vorzugsweise aus der Gruppe, bestehend aus Selen-, Brom- und Iod-enthaltenden Aminosäure-Analoga ausgewählt. Besonders bevorzugt wird in diesem Fall das Aminosäure-Analogon von Tryptophan aus der Gruppe, bestehend aus β-Selenolo[3,2-b]pyrrolyl-L-alanin, β-Selenolo[2,3-b]pyrrolyl-L-alanin, 4-Bromo-Tryptophan, 5-Bromo-Tryptophan, 6-Bromo-Tryptophan, 4-Iodo-Tryptophan, 5-Iodo-Tryptophan und 6-Iodo-Tryptophan ausgewählt.

Falls bei einer alternativen Ausführungsform der vorliegenden Erfindung die Bestimmung der Phasen der Streuwellen (Strukturfaktoren) in Schritt c) des vorstehend beschriebenen erfindungsgemäßen Verfahrens nach der Methode der multiplen Schweratomderivate erfolgt, wird das Aminosäure-Analogon von Tryptophan vorzugsweise ausgewählt aus der Gruppe, bestehend aus Schwefel-, Tellur-, Brom- und Iod-enthaltenden Aminosäure-Analoga. Besonders bevorzugt wird in diesem Fall das Aminosäure-Analogon von Tryptophan ausgewählt aus der Gruppe, bestehend aus β-Thieno[3,2-b]pyrrolyl-L-alanin, β-Thieno[2,3-b]pyrrolyl-L-alanin, β-Tellurolo[3,2-b]pyrrolyl-L-alanin, β-Tellurolo[2,3-b]pyrrolyl-L-alanin, 4-Bromo-Tryptophan, 5-Bromo-Tryptophan, 6-Bromo-Tryptophan, 4-Iodo-Tryptophan, 5-Iodo-Tryptophan und 6-Iodo-Tryptophan.

Ein weiterer Aspekt der vorliegenden Erfindung ist die Verwendung der vorstehend beschriebenen erfindungsgemäßen Proteine, Peptide bzw. peptidischen Leitstrukturen zur Bestimmung der Phasen der Streuwellen (Strukturfaktoren) bei der Kristallstrukturanalyse.

Ferner können erfindungsgemäß die Chalkogen- und/oder Halogen-enthaltenden Aminosäure-Analoga von Tryptophan zur Markierung von Proteinen, Peptiden oder peptidischen Leitstrukturen für Fluoreszenz- und/oder UV- und/oder NMR-spektroskopische Untersuchungen verwendet werden. Für NMR-spektroskopische Untersuchungen werden besonders bevorzugt ⁷⁷Selen-enthaltende Aminosäure-Analoga von Tryptophan verwendet.

Bei einem weiteren Aspekt der vorliegenden Erfindung kann das erfindungsgemäße Verfahren zur Markierung von Proteinen, Peptiden oder peptidischen Leitstrukturen in der Proteomanalyse verwendet werden. Ferner kann das zuletzt genannte Verfahren erfindungsgemäß zur Identifizierung und Charakterisierung von Targetproteinen verwendet werden.

Ein weiterer Gegenstand der vorliegenden Erfindung betrifft pharmazeutische Zusammensetzungen, die als Wirkstoff mindestens ein Chalkogen-enthaltendes Aminosäure-Analogon von Tryptophan, eingebaut in einen Träger, ausgewählt aus einem Protein, einem Peptid oder einer peptidischen Leitstruktur, umfassen. Vorzugsweise ist das Chalkogen Schwefel. Besonders bevorzugt ist das Aminosäure-Analogon von Tryptophan β-Thieno[3,2-b]pyrrolyl-L-alanin oder β-Thieno[2,3-b]pyrrolyl-L-alanin.

Bei einer bevorzugten Ausführungsform ist der Träger in der pharmazeutischen Zusammensetzung, der ein Chalkogen-enthaltendes Analogon von Tryptophan umfasst, ein Protein, ein Peptid bzw. eine peptidische Leitstruktur, das eine mit dem entsprechenden Wildtyp des Proteins, des Peptids bzw. der peptidischen Leitstruktur weitgehend identische dreidimensionale Struktur aufweist.

Vorzugsweise ist der Träger ein humanes Peptid oder Protein, besonders bevorzugt ein rekombinantes Peptid oder Protein, ganz besonders bevorzugt ein gewebespezifisches Peptid oder Protein.

Schließlich ist es ein weiterer Aspekt der vorliegenden Erfindung, dass die vorstehend beschriebenen Proteine, Peptide bzw. peptidischen Leitstrukturen erfindungsgemäß zur Herstellung eines Arzneimittels, welches als Wirkstoff ein Chalkogen-enthaltendes Aminosäure-Analogon von Tryptophan umfasst, verwendet werden, vorzugsweise zur Herstellung eines Halluzinogens oder alternativ zur Herstellung eines Serotonin-Agonisten.

Es ist jetzt überraschenderweise erstmals gelungen, Proteine, Peptide und peptidische Leitstrukturen bereitzustellen, die mindestens ein Chalkogen-enthaltendes Aminosäureanalogon von Tryptophan und/oder ein Halogen-enthaltendes Aminosäureanalogon von Tryptophan umfassen.

Das Chalkogen ist vorzugsweise Schwefel-, Selen- oder Tellur und besonders bevorzugt Selen. Das Halogen ist vorzugsweise Brom oder Iod.

Vorzugsweise ist das dem Indolring von Tryptophan entsprechende Ringsystem des Aminosäureanalogons von Tryptophan, das in dem erfindungsgemäßen Protein, dem erfindungsgemäßen Peptid bzw. der erfindungsgemäßen peptidischen Leitstruktur enthalten ist, aromatisch. Beispielsweise kann das dem Indolring von Tryptophan entsprechende Ringsystem zwei kondensierte Fünfringe umfassen, wobei sich eine NH-Gruppe in dem einem Fünfring befindet, während das Chalkogen, beispielsweise S, Se oder Te, in dem anderen Fünfring eingebaut ist.

Bei einer besonders bevorzugten Ausführungsform der vorliegenden Erfindung wird das Aminosäureanalogon von Tryptophan, das in dem erfindungsgemäßen Protein, dem erfindungsgemäßen Peptid bzw. der erfindungsgemäßen peptidischen Leitstruktur enthalten ist, aus den folgenden Verbindungen ausgewählt: β-Selenolo[3,2-b]pyrrolyl-L-alanin, β-Selenolo[2,3-b]pyrrolyl-L-alanin, β-Thieno[3,2-b]pyrrolyl-L-alanin, β-Thieno[2,3-b]pyrrolyl-L-alanin, β-Tellurolo[3,2-b]pyrrolyl-L-alanin und β-Tellurolo[2,3-b]pyrrolyl-L-alanin.

Bei einer weiteren bevorzugten Ausführungsform ist das dem Indolring von Tryptophan entsprechende Ringsystem des Aminosäureanalogons von Tryptophan mit Halogenen substituiert. Beispiele hierfür sind 4-Bromo-Tryptophan, 5-Bromo-Tryptophan, 6-Bromo-Tryptophan sowie die entsprechenden Jod-, Fluor- und Chlor-Verbindungen.

Das Aminosäureanalogon von Tryptophan kann in ein Protein, ein Peptid oder eine peptidische Leitstruktur eingebaut werden, indem es eine wie vorstehend beschriebene verwandte kanonische Aminosäure in dem Protein, dem Peptid bzw. der peptidischen Leitstruktur ersetzt.

Vorzugsweise werden alle Tryptophanreste der Sequenz eines nativen Proteins, eines nativen Peptids oder einer nativen peptidischen Leitstruktur durch Chalkogen-Analoga von Tryptophan ersetzt.

Die Biosynthese von Proteinen mit elektronenreichen Atomspezies unter Beibehaltung ihrer dreidimensionalen Struktur im kristallinen Zustand durch rationelles Design ist eine der vielversprechendsten Ansätze für die Protein-Kristallstrukturanalyse in den letzten Jahren. Beispielsweise ist bekannt, dass der Ersatz von Schwefel durch Selen in Methioninresten von Proteinen ein sehr effektives Resonanzzentrum für die anomale Streuung von Röntgenstrahlen darstellt. Anomale Streuung ist die Hauptvoraussetzung für die MAD-Methode, die mittlerweile zur Lösung des Phasenproblems in der Kristallographie weitverbreitet ist (Hendrickson, Science 1991, 254, 51-58).

Selenomethionin und Fluorleucin sind die einzig bekannten nicht-kanonischen Aminosäuren, die aufgrund ihrer moderaten Toxizität das Wachstum von auxotrophen *E.coli*-Stämmen *in vivo* in definiertem Minimalmedium unterstützen. Andere nicht-kanonische Aminosäuren, die noch schwerere Atome als Selen enthalten, wie beispielsweise Telluromethionin, sind hochtoxisch und lassen kein zelluläres Wachstum zu. So sind nahezu alle Aminosäuren außer den zwanzig kanonischen Aminosäuren effiziente Inhibitoren des zellulären Wachstums.

Mit Hilfe des SPI-Verfahrens war es möglich, als Methionin-Analogon Telluromethionin in verschiedene Proteine zu inkorporieren (Budisa et al., J. Mol. Biol. 1997, 270, 616-623). Aufgrund der hohen Toxizität für die Wirtszelle und einer extremen Oxidationsempfindlichkeit hat Telluromethionin keine breite und allgemeine Akzeptanz in der Proteinkristallographie erlangt. Ferner besteht ein grundsätzlicher Nachteil des Einbaus von Methionin-Analoga in Proteine zur Erleichterung der Lösung des Phasenproblems bei der Kristallographie darin, dass die Aminosäure Methionin in Proteinsequenzen relativ häufig vorkommt und somit die Lokalisierung der Resonanzzentrum erschwert ist.

Um diese Beschränkungen zu umgehen, wurde erfindungsgemäß die kanonische Aminosäure Tryptophan als Target für einen derartigen Ersatz durch ein Aminosäure-Analogon ausgewählt, da beispielsweise ein in das aromatische System integriertes Chalkogenatom, insbesondere ein Schwefel- oder Selenatom, ein stabiles und planares Aminosäure-Analogon ergibt, das als Tryptophan-Ersatz dienen kann. Ein weiterer Vorteil ist, dass Tryptophan weitaus weniger häufig in globulären Proteinen vorliegt, nämlich nur einen Anteil von etwa 1 % aller Aminosäurereste von globulären Proteinen aufweist (McCaul und Ludescher, Photochem. Photobiol. 1999, 70, 166-171). Aufgrund dieses seltenen Vorkommens in globulären Proteinen liefert die Substitution eines Tryptophanrests durch ein Chalkogen-enthaltendes Aminosäure-Analogon und/oder ein Halogen-enthaltendes Aminosäure-Analogon, vorzugsweise ein Selen-enthaltendes Aminosäure-Analogon von Tryptophan, ein effektives ortsspezifisches intrinsisches Resonanzzentrum für die Proteinstrukturbestimmung durch das MAD-Verfahren. Die im Rahmen der vorliegenden Erfindung beschriebenen Chalkogen-enthaltenden und Halogen-enthaltenden Aminosäure-Analoga von Tryptophan stellen eine wertvolle Ergänzung für die Phasenberechnungen durch das MAD-Verfahren dar, da diese Aminosäuren einige zusätzliche Vorteile bringen. Diese Vorteile werden im Folgenden hinsichtlich der relativen Häufigkeit und der bevorzugten topologischen Positionen von Methionin- und Tryptophan-Resten in Proteinen dargestellt.

Ein Nachteil der Verwendung von Selenomethionin in der Diffraktionsanalyse von derivatisierten Kristallen großer Proteine entsteht dadurch, dass die Anzahl an Selenenthaltenden Resten zu groß sein kann, um erfolgreich durch direkte Mehtoden oder Patterson-Methoden bestimmt zu werden. In diesem Zusammenhang kann das erfindungsgemäß in Proteine, Peptide oder peptidische Leitstrukturen inkorporierte Chalkogen-enthaltende und/oder Halogen-enthaltende Aminosäure-Analogon von Tryptophan eine vorteilhafte Alternative sein, da der UGG-Kodon von Tryptophan im allgemeinen weit weniger häufig als der AUG-Kodon von Methionin in Proteingenen ist (McCaul und Ludescher, 1999, siehe oben).

In Sequenzvergleichen werden ferner üblicherweise Methionin, Leucin und Isoleucin ähnliche Hydrophobizitäten zugeordnet. Die Methionin-Seitenkette ist jedoch wesentlich flexibler als die verzweigten und starren Seitenketten von Leucin und Isoleucin. Methionin ist in Proteinstrukturen sowohl in hydrophoben Wechselwirkungen als auch in Wasserstoffbrückenbindungen involviert. Ferner besitzt Methionin einen Anteil von etwa 1,5 % an allen Resten in Proteinen bekannter Struktur und ist üblicherweise Lösungsmittelunzugänglich, da nur 15 % aller Methioninreste an der Oberfläche angeordnet sind (Rose et al., Science 1985, 229, 834-838).

Durch die Methionin-Flexibilität, insbesondere von solchen Methioninen, die sich an der Proteinoberfläche befinden, können somit in manchen Fällen Probleme hinsichtlich der Atommobilität entstehen, was sich in den kristallographischen B-Faktoren äußert. Dagegen sind aromatische Seitenketten, wie Tryptophan, weit weniger in innere Proteinbewegungen involviert als kleinere Aminosäuren wie Methionin, da sie häufig ein Teil des starren Proteininneren darstellen. Aromatische Reste wie Tryptophan bilden häufig ein Netzwerk von drei oder mehreren wechselwirkenden Seitenketten in Proteinen, weshalb derartige Protein-Core-Bereiche üblicherweise starre Bereiche frei von Flexibilität darstellen.

Deshalb stellen in Fällen, in denen Methionin entweder aufgrund der relativ hohen Häufigkeit oder der kristallographischen B-Faktoren für die Diffraktionsanalyse weniger gut geeignet ist, Chalkogen- und/oder Halogen-enthaltende Aminosäure-Analoga von Tryptophan eine wertvolle Alternative dar.

Allerdings können sich auch Tryptophan-Reste an der Oberfläche von Proteinstrukturen befinden. Dies beruht auf der Tatsache, dass Tryptophan zwei Eigenschaften vereinigt, nämlich Hydrophobizität (durch die Zusammensetzung aus Kohlenwasserstoffeinheiten) und Polarität (d.h. die Fähigkeit zur Bindung von Ionen durch Kationen-π-Wechselwirkungen, die häufig als sich gegenseitig ausschließend angenommen werden. Diese Kombination von Eigenschaften macht Tryptophan zu einem geeigneten Aminosäurerest sowohl für das Proteininnere als auch für eine Zellmembran. Somit können je nach Bedarf für die Berechnung der Phasen durch die MAD-Methode Methionin- und/oder Tryptophan-Analoga in die Proteine inkorporiert werden, um Proteinderivate zu erzeugen, die geeignete Diffraktions-Markierungen enthalten.
Durch die Inkorporation von Chalkogen- und/oder Halogen-enthaltenden Aminosäure-Analoga von Tryptophan in Proteine basierend auf dem SPI-Verfahren, das weit weniger aufwendig ist als ortsspezifische Mutagenese-Protokolle, ist die Routinedurchführung dieser MAD-Experimente leicht zugänglich. Aufgrund der Stabilität dieser Substanz in Routinefermentations- und Kristallisationsexperimenten und der relativ niedrigen Häufigkeit von Tryptophan-Resten in Proteinen ist die *in vivo*-Inkorporation von Chalkogen- und /oder Halogen-enthaltenden Aminosäure-Analoga von Tryptophan, insbesondere von Selenenthaltenden Tryptophan-Analoga, der Selenomethionin-Inkorporation in Proteine sogar überlegen.

Das Phänomen der anomalen Streuung beruht auf der Tatsache, dass, wenn die Energie der Röntgenstrahlen im Bereich der Energie eines Elektronenübergangs eines gebundenen Atomorbitals liegt, eine Resonanzbedingung erfüllt ist, die die Beschleunigung der Elektronen verstärkt und die Streuung stört. Röntgenstrahlen in Standard-Diffraktionsexperimenten mit einer Energie und Wellenlänge im Bereich von 10.000 eV bzw. 1 Å führen nicht zu Elektronenübergängen bei den leichten Atomen in biologischen Makromolekülen, wie Wasserstoff, Kohlenstoff, Stickstoff, Sauerstoff, Schwefel und Phosphor. Die Resonanzkomponente ist in solchen Fällen vernachlässigbar. Die "anomale" Resonanzstreuung ist auf schwerere Atome beschränkt, wie in der folgenden Tabelle gezeigt wird.

**Tabelle 1**

| Atom | eV | Å |
|---|---|---|
| Se | 12.658 | 0,979 |
| Br | 13.474 | 0,920 |
| I | 33.169 | 0,374 |
| Te | 31.814 | 0,390 |
| Sb | 30.491 | 0,407 |

Somit wird zur Bestimmung der Phasen der Strukturfaktoren nach der MAD-Methode das Aminosäure-Analogon von Tryptophan vorzugsweise ausgewählt aus der Gruppe, bestehend aus Selen-, Brom- und Iod-enthaltenden Aminosäure-Analoga. Besonders bevorzugt wird Selen oder Brom in das Tryptophan-Analogon eingebaut. Beispiele für besonders bevorzugte Aminosäure-Analoga von Tryptophan sind β-Selenolo[3,2-b]pyrrolyl-L-alanin, β-Selenolo[2,3-b]pyrrolyl-L-alanin, 4-Bromo-Tryptophan, 5-Bromo-Tryptophan, 6-Bromo-Tryptophan, 4-Iodo-Tryptophan, 5-Iodo-Tryptophan und 6-Iodo-Tryptophan.

Durch die vorstehend beschriebenen intrinsischen Eigenschaften der Aminosäure Tryptophan hinsichtlich ihrer relativen Häufigkeit in Proteinen und der relativ geringen Flexibilität ist der Einbau von Tryptophan-Analoga in Proteine, Peptide oder peptidischen Leitstrukturen auch zur Kristallstrukturbestimmung nach der Methode der multiplen Schweratomderivate besonders geeignet. In diesem Fall werden die Tryptophan-Analoga vorzugsweise ausgewählt aus der Gruppe, bestehend aus Schwefel-, Tellur-, Brom- und Iod-enthaltenden Aminosäure-Analoga. Beispiele für besonders bevorzugte Aminosäure-Analoga von Tryptophan sind β-Thieno[3,2-b]pyrrolyl-L-alanin, β-Thieno[2,3-b]pyrrolyl-L-alanin, β-Tellurolo[3,2-b]pyrrolyl-L-alanin, β-Tellurolo[2,3-b]pyrrolyl-L-alanin, 4-Bromo-Tryptophan, 5-Bromo-Tryptophan, 6-Bromo-Tryptophan, 4-Iodo-Tryptophan, 5-Iodo-Tryptophan und 6-Iodo-Tryptophan.

Ferner kann ein Peptid oder eine peptidische Leitstruktur in Form eines Peptidinhibitors erfindungsgemäß mit einem für MAD-Messungen geeigneten Aminosäureanalogon von Tryptophan versehen werden, um es mit einem Rezeptor in Form eines Proteins zu cokristallisieren.

UV-Absorption und Fluoreszenz sind Eigenschaften von Proteinen, die auf der Gegenwart aromatischer Aminosäuren, insbesondere der Gegenwart von Tryptophan, in der Struktur beruhen. Eine Erweiterung des Aminosäure-Repertoirs für die Proteinbiosynthese *in vivo* ermöglicht die Synthese von Proteinen mit den vorstehend beschriebenen Chalkogen-enthaltenden und/oder Halogen-enthaltenden Analoga von Tryptophan, wodurch neue spezielle spektrale Eigenschaften in die Strukturen der Proteinmutanten eingebracht werden.

Obwohl Tryptophan als Hauptchromophor in Proteinen eine intrinsische Sonde darstellt, ist dieser Aminosäurerest für die Untersuchung von Protein/Protein- oder Protein-/Nukleinsäure-Wechselwirkungen mit UV- bzw. Fluoreszenz-Spektroskopie weniger geeignet, da die Absorptionsspektren von Nukleinsäuren mit denen von Tryptophan überlappen und somit die Zuordnung des spektralen Beitrags von Tryptophanresten zu dem Gesamtsignal verhindern. Ähnliche Schwierigkeiten entstehen, wenn Protein/Protein-Wechselwirkungen untersucht werden, da viele wechselwirkende Proteine in Protein-Komplexen Tryptophanreste enthalten und somit deren Absorptions- und Fluoreszenzsignale mehr oder weniger nicht unterscheidbar sind (Ross et al., Methods Enzymol. 1997, 278, 151 - 190).

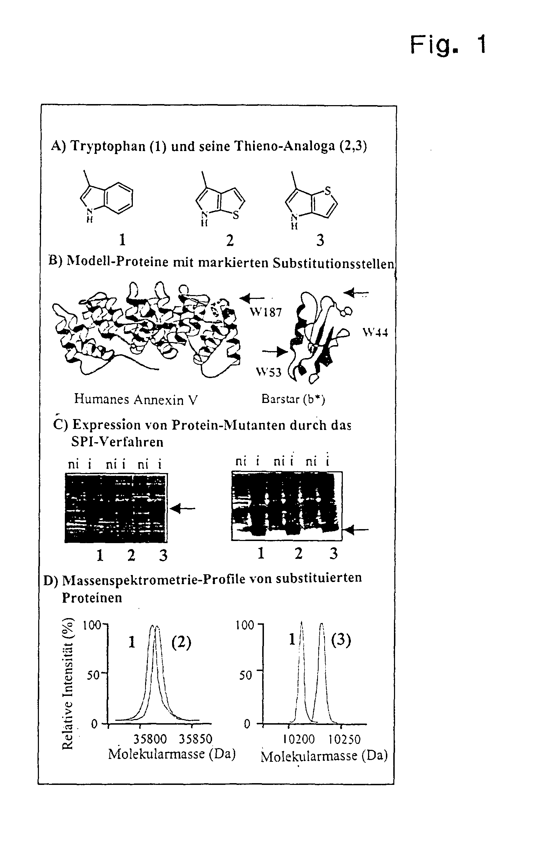

Ein üblicher Ansatz zur Untersuchung der funktionellen Rolle von Tryptophan in Proteinen ist die Verwendung von ortsspezifischer Mutagenese. Dabei werden in den meisten Fällen Tryptophan-Reste in Phenylalanin mutiert, um die strukturellen Störungen durch Ersatz einer aromatischen planaren Gruppe mit einer anderen zu minimieren. Diese Strategie ist jedoch begrenzt einsetzbar, da Tryptophanreste häufig wesentlich für die strukturelle Integrität und Funktionalität von Proteinen sind und deshalb nicht durch irgendeine der übrigen 19 kanonischen Aminosäuren ersetzt werden können, wie es beispielsweise der Fall bei W187 in AnnexinV und bei W53 in Barstar ist (siehe Abbildung 1). Selbst wenn ein derartiger Ersatz möglich ist, könnten lokale strukturelle Störungen die spektralen Beiträge der verbleibenden Chromophore stören, da die ähnlichsten kanonischen Gegenstücke Phenylalanin und Tyrosin immer relativ große Veränderungen sowohl hinsichtlich der Größe als auch hinsichtlich der Ladung mit sich bringen.

Durch den Einbau von nicht-kanonischen Tryptophan-Analoga werden geringere Veränderungen erwartet, was die Interpretation von experimentellen Daten hinsichtlich spektraler Überlappung, besserer Empfindlichkeit auf geringe Störungen der Umgebung der substituierten aromatischen Seitenketten und neuer spektraler Fenster erleichtern könnte.

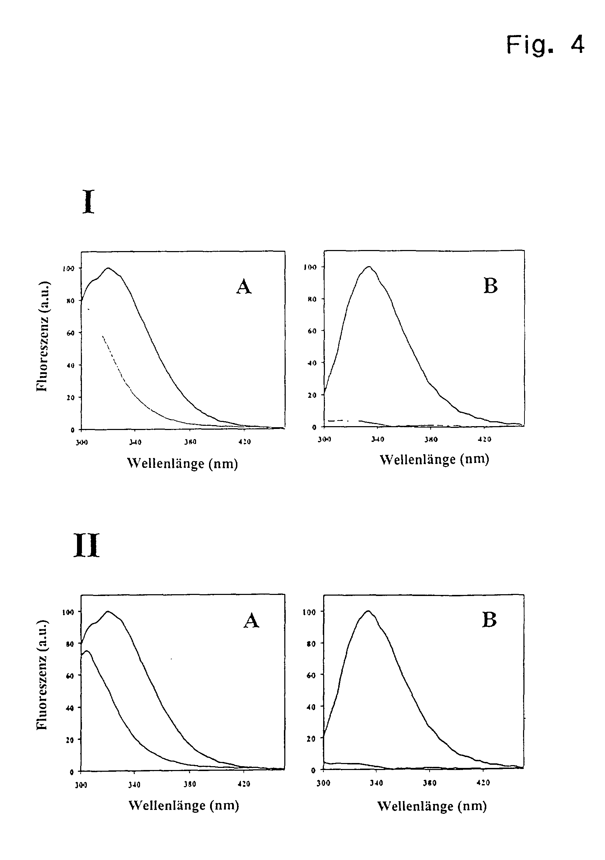

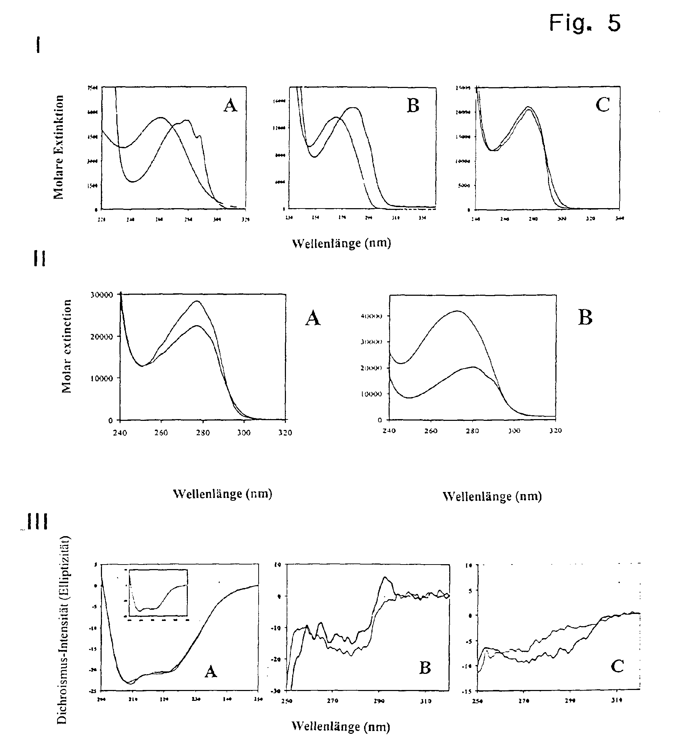

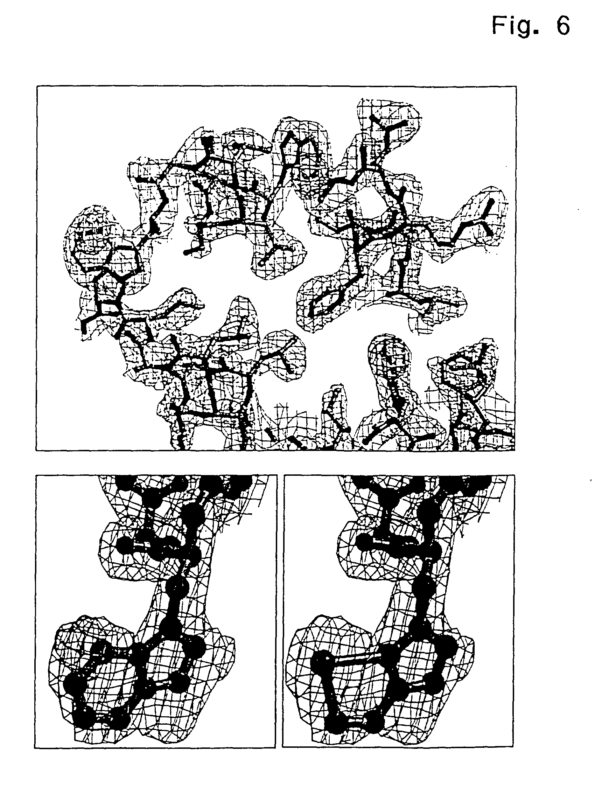

Durch den Einbau von β-(Thienopyrrolyl)-alaninen in Proteine wurden erfindungsgemäß zwei bemerkenswerte spektroskopische Eigenschaften erhalten, nämlich zum einen veränderte UV-Absorptionsprofile, insbesondere wenn der Beitrag von Tryptophan wie im Fall der Barstar-Mutante dominant ist (siehe Abbildung 5 II), und zum anderen ein effizientes statisches Fluoreszenz-Quenching (siehe Abbildung 4). Bislang war mit 4-Fluoro-Tryptophan lediglich ein nicht-fluoreszierendes Tryptophan-Analogon bekannt, das in vivo in Proteine eingebaut wurde (Bronskill und Wong, Biochem. J. 1988, 249, 305 - 308). Somit wird durch die vorstehende Erfindung das Repertoire an "ruhigen" Fluorophoren als Proteinbausteine für die in-vivo-Translation erweitert und damit dem Bedarf bei der Untersuchung von Proteinen/DNA-Wechselwirkungen oder Protein-Komplexen Rechnung getragen.

Beispielsweise zeigen die Fluoreszenz-Emissionsspektren von Tpa- und Sep-Annexin V bzw-Barstar b*, dass die Tryptophan-Emission bei 320 nm unterdrückt ist und die Beiträge der Tyrosinreste die Maxima der Emissionsspektren bilden (siehe Abbildung 4). Somit ist eine Differenzierung in Gegenwart anderer Tryptophan-haltiger Proteine, Peptide oder peptidischer Leitstrukturen möglich. Das Fehlen der Tryptophan-Emission ist auf die strukturellen Unterschiede der Chalkogen-enthaltenden Aminosäureanaloga zu Tryptophan zurückzuführen, die keine Fluorophore mehr darstellen. Der Einbau der vorstehend beschriebenen Aminosäureanaloga von Tryptophan bietet für die Fluoreszenzspektroskopie neue Möglichkeiten für die detaillierte Untersuchung von lokalen strukturellen und/oder dynamischen Eigenschaften von Proteinen, Peptiden, peptidischen Leitstrukturen und makromolekularen Komplexen.

Ebenso weisen die UV-Absorptionsspektren der Chalkogen-enthaltenden Analoga von Tryptophan charakteristische Maxima auf, die bei der Inkorporation in Proteine, Peptide oder peptidische Leitstrukturen signifikante Veränderungen ihrer UV-Absorptionseigenschaften hervorrufen (siehe Abbildung 5).

Gegenstand der vorliegenden Erfindung ist somit auch ein Verfahren zur Markierung von Proteinen, Peptiden oder peptidischen Leitstrukturen für UV- und/oder Fluoreszenz-spektroskopische Untersuchungen, bei dem mindestens ein Chalkogen-enthaltendes Aminosäureanalogon von Tryptophan und/oder mindestens ein Halogen-enthaltendes Aminosäureanalogon von Tryptophan in ein Protein, ein Peptid oder eine peptidische Leitstruktur eingebaut wird, wobei das Chalkogen aus der Gruppe, bestehend aus Schwefel, Selen und Tellur ausgewählt wird, und das Halogen aus der Gruppe, bestehend aus Brom und Iod ausgewählt wird.

Alle Benzol-basierten Aminosäuren (Phenylalanin, Tyrosin, Tryptophan) weisen starke Quadropolmomente auf, die aus der nicht-sphärischen Ladungsverteilung resultieren. Somit führt die Einführung von Heteroatomen in diese Systeme zu neuen Eigenschaften.

Beispielsweise hat das Schwefelatom in Thiophen ein ungepaartes Elektronenpaar in einem p-Orbital, das mit den Kohlenstoff-Kohlenstoff-Doppelbindungen konjugiert ist. Ferner besitzt Schwefel im Gegensatz zu Kohlenstoff, Stickstoff und Sauerstoff freie d-Orbitale in der äußeren Schale und kann deswegen als Elektronenakzeptor wirken. Die kondensierten Pyrrole sind einer Wechselwirkung mit benachbarten Ladungen durch die Induktion von Dipolen in dem System sowie durch zusätzliche Dispersionskräfte, Polarisierbarkeit, Exciplex-Bildung, Resonanzenergietransfer oder durch die Bildung von Charge-Transfer-Komplexen leichter zugänglich. Andere Eigenschaften, die sich von Benzol unterscheiden, umfassen eine verstärkte Hyperpolarisierbarkeit und Unterschiede in aromatischen Delokalisierungsenergien (Benzol: 36 kcal/mol, Thiophen: 29 kcal/mol, Thiazol: 25 kcal/mol) (Bird, Tetrahedron 1985, 41, 1409 - 1414).

Es wird deshalb erwartet, dass der Ersatz des Benzolrings in dem Indolring von Tryptophan durch Thiophen zu veränderten Wechselwirkungen führt, die verantwortlich für die Struktur von biologischen Makromolekülen und für die Vermittlung von Prozessen wie Rezeptor-Ligand-Wechselwirkungen, Enzym-Substrat-Bindung und Antigen-Antikörper-Erkennung sind. Dies wurde in Untersuchungen von Struktur-Aktivitäts-Beziehungen mit dem Enzym Tryptophan-Indol-Lyase gezeigt. Für [2,3]Tpa war der k_{cat}/K_{M}-Wert eine Größenordnung höher (8,6 x 103 M⁻¹s⁻¹) als der von [3,2]Tpa (1,2 x 10³ M⁻¹s⁻¹). Dies wurde den Unterschieden in der elektronischen Zusammensetzung zugeordnet (Sloan und Phillips, Biochemistry 1996, 35, 16165 - 16173).

Thiophen-basierte Substanzen sind von großem Interesse in der Materialwissenschaft, da sie eine Reihe nützlicher Eigenschaften wie eine verbesserte optische Transparenz oder eine gute thermische Stabilität aufweisen, die aus den induktiven Effekten des elektronenreichen Schwefelatoms resultieren (Kothakota et al., J. Am. Chem. Soc. 1995, 117, 536 - 537, Breitung et al., J. Am. Chem. Soc. 2000, 122, 1154 - 1160).

Der Einbau von Chalkogen- und/oder Halogen-enthaltenden Analoga von Tryptophan in Peptide, peptidische Leitstrukturen und Proteine kann durch synthetische und semisynthetische Methoden sowie durch rekombinante DNA-Technologie erfolgen. Während mit Methoden der chemischen Synthese Peptide, peptidische Leitstrukturen und kleinere Proteine mit einer Molekülmasse von < 10 kDa zugänglich sind, wird für größere Proteine ein wirksames rekombinantes Expressionssystem wie beispielsweise das bakterielle T7-Promotor-Polymerasesystem bevorzugt.

Der Einbau des Aminosäureanalogons kann durch chemische Synthese, beispielsweise Festphasenpeptidsynthese oder aber vorzugsweise in vivo in einer Wirtszelle oder in einem in vitro-Translationssystem erfolgen. Die Bioinkorporation nicht kanonischer Aminosäureanaloga in Proteine durch eine in vivo-Methode ist gegenüber der chemischen Synthese bevorzugt, da bei der Bioinkorporation ein selektiver Einbau von L-Enantiomeren der Aminosäureanaloga erfolgt, so dass mögliche unerwünschte Nebenwirkungen aufgrund des Vorhandenseins von D-Enantiomeren ausgeschlossen werden können. Bei der chemischen Synthese können hingegen auch D-Enantiomere eingebaut werden, so dass - um dies zumindest weitgehend zu verhindern - eine kostspielige und aufwendige Aufreinigung der Ausgangsmaterialien erforderlich ist. Selbst dann können jedoch Verunreinigungen durch D-Enantiomere nicht vollständig ausgeschlossen werden.

Die Bioinkorporation der Aminosäureanaloga erfolgt bei der vorliegenden Erfindung vorzugsweise durch das Selektionsdruck-Einbau-Verfahren (SPI-Verfahren, selection pressure incorporation). Bei der SPI-Methode werden Zellen, insbesondere Bakterienzellen, in einem definierten Medium mit einer limitierten Konzentration der jeweils zu ersetzenden kanonischen Aminosäure kultiviert, wobei die Transkription des Gens, das für das erfindungsgemäße Protein kodiert, abgeschaltet bleibt. Nach Verbrauch der kanonischen Aminosäure und dem Beginn der stationären Wachstumsphase wird die kanonische Aminosäure durch das entsprechende Aminosäureanalogon ersetzt. Eine Induktion der Biosynthese des rekombinanten Targetproteins führt zur Herstellung eines Proteins, welches ausschließlich das nicht kanonische Aminosäureanalogon anstelle der kanonisch vorkommenden Aminosäure enthält. Mit dieser SPI-Methode wurden bereits erfolgreich Aminosäureanaloga wie Selenomethionin, Ethionin, Telluromethionin, Selenocystein und Norleucin in diverse Modellproteine eingebaut, wobei als Wirtszelle ein für die jeweils zu substituierende Aminosäure auxotropher *E. coli*-Stamm und ein T7-Promotor/Polymerase-Expressionssystem verwendet wurde (Budisa et al., Eur. J. Biochem. 230, 788-796 (1995); Karnbrock et al., J. Am. Chem. Soc. 118, 913-914 (1996) und Besse et al., Biol. Chem. 378, 211-218 (1997)). Im Rahmen der vorliegenden Anmeldung wird nun erstmals beschrieben, wie die SPI-Methode zur Bioinkorporation eines Chalkogen-Analogons, wobei das Chalkogen aus der Gruppe, bestehend aus Schwefel, Selen und Tellur, ausgewählt ist, bzw. eines Halogen-Analogons, wobei das Halogen aus der Gruppe, bestehend aus Brom und Jod, ausgewählt ist, von Tryptophan anstelle der kanonischen, genetisch kodierten Aminosäure Tryptophan in ein humanes Protein verwendet werden kann.

Bei einer weiteren Ausführungsform der vorliegenden Erfindung wird mindestens ein Chalkogen- und/oder Halogen-enthaltendes Analogon von Tryptophan zusätzlich zu der nativen Sequenz in das Protein, das Peptid bzw. die native peptidische Leitstruktur eingebaut.

Vorzugsweise wird auf DNA-Ebene durch Klonierung und/oder Mutagenese eine für die entsprechende Aminosäure kodierende Sequenz angefügt, wobei der Einbau des mindestens einen Aminosäureanalogons von Tryptophan vorzugsweise durch selektiven Druck, d.h. besonders bevorzugt durch das SPI-Verfahren, erfolgt. Andere Verfahren, wie z.B. ein in vitro-Einbau, können ebenfalls verwendet werden, weisen allerdings die beschriebenen Nachteile auf.

Bei Verwendung von Wirtszellen umfasst der Einbau des Aminosäureanalogons vorzugsweise folgende Schritte:
a) Bereitstellen einer Wirtszelle mit einer für das Targetprotein kodierenden Nukleinsäure in operativer Verknüpfung mit einer regulierbaren Expressionskontrollsequenz,
b) Kultivieren der Wirtszelle unter Bedingungen, bei denen die Transkription der für das Peptid oder Protein kodierenden Nukleinsäure weitgehend abgeschaltet ist,
c) Zugeben des Aminosäureanalogons von Tryptophan, und
d) Induzieren der Expression des Peptids oder Proteins.

Vorzugsweise verwendet man als Wirtszelle eine prokaryontische Zelle, insbesondere eine *E. coli*-Zelle oder eine eukaryontische Zelle wie beispielsweise eine Hefezelle. Weiterhin ist bevorzugt, dass man eine Wirtszelle verwendet, die für die durch das Aminosäureanalogon zu ersetzende Aminosäure auxotroph ist, und bei Schritt a) des vorstehenden Verfahrens die zu ersetzende Aminosäure dem Medium zusetzt. Solche für ein oder mehrere Aminosäuren auxotrophe Wirtszellen sind wohl bekannt und können ggf. durch Mutation einzelner für die Biosynthese bestimmter Aminosäuren verantwortlicher Gene ohne weiteres hergestellt werden.

Für das erfindungsgemäße Verfahren können grundsätzlich beliebige regulierbare Expressionskontrollsequenzen, beispielsweise chemisch regulierbare Expressionskontrollsequenzen, wie etwa das lac-, das trp- oder das tac-Expressionssystem, oder temperaturregulierbare Expressionssysteme, wie das λ_{PL}-Expressionssystem, eingesetzt werden. Vorzugsweise wird jedoch als regulierbare Expressionskontrollsequenz ein Bakteriophagen-Promotor in Kombination mit einer Wirtszelle verwendet, die ein Gen für eine, den Promotor erkennende DNAabhängige RNA-Polymerase enthält, wobei dieses Polymerase-Gen wiederum in operativer Verknüpfung mit einer weiteren regulierbaren Expressionskontrollsequenz, beispielsweise einem temperaturabhängigen Lambda-Promotor steht. Besonders bevorzugt verwendet man einen T7-Promotor in Kombination mit einem T7-RNA-Polymerase-Gen. Dieses Expressionssystem wird von Studier und Moffat (J. Mol. Biol. 189, 113-130 (1986)) und Studier et al. (Methods Enzymol. 185, 60-89 (1991)) beschrieben.

Im Folgenden werden wichtige Aspekte und Perspektiven der Aminosäureanaloga von Tryptophan als allgemeine nicht-invasive Sonden für die erfindungsgemäßen Proteine, Peptide und peptidischen Leitstrukturen insbesondere im Rahmen des SPI-Verfahrens diskutiert.

Zunächst ist es erforderlich, dass die Markierung des Targetproteins spezifisch gegenüber allen zellulären Proteinen des Expressionswirts ist. Dabei ist es nicht entscheidend, dass die Zellen des Expressionswirts wachsen, sondern dass die Zellen das Targetprotein - und zwar idealerweise ausschließlich oder im wesentlichen ausschließlich - exprimieren. Beispielsweise wird in dem T7-Promotor/Polymerasesystem das zelluläre Wachstum des Wirts nach der Induktion der Proteinsynthese nicht mehr unterstützt, d.h., die gesamte zelluläre Maschinerie steht in Funktion der Targetproteinherstellung (S. Beiboer et al., siehe oben). Aber selbst dann kann nicht ausgeschlossen werden, dass andere Proteine bis zu einem gewissen Grad während der Proteinbiosynthese ebenfalls mit dem Chalkogen-Analogon bzw. Halogen-Analogon von Tryptophan markiert werden, wie die T7-Polymerase, die ebenfalls durch die Induktion hergestellt wird. In diesem Fall kann eine getrennte chromatographische Charakterisierung dieses Proteins hilfreich sein. Alternativ kann ein Wechsel zu einem anderen, beispielsweise auf tac-Promotoren basierenden Expressionssystem vollzogen werden.

Zweitens ist es erforderlich, dass die Markierung des Proteins nicht-invasiv ist. Das heißt, es ist erwünscht, so weit wie möglich die native Sekundär- und Tertiärstruktur sowie die Stabilität und Aktivität des Proteins beizubehalten.

Ein großer Vorteil des SPI-Verfahrens ist, dass die grundlegenden Isolierungs- und Reinigungsprotokolle, die für die Wildtyp-Proteinformen entwickelt und optimiert wurden, für die Proteinmutanten ohne besondere Maßnahmen oder Modifikationen übernommen werden können.

Erfindungsgemäß wird somit ein Verfahren bereitgestellt, das als einfache, kostengünstige, reproduzierbare und zuverlässige Methode die Erkennung von Targetproteinen, -peptiden und peptidischen Leitstrukturen beschleunigt, was im kommenden Zeitalter der "Proteomics" von großem praktischem Nutzen und Potential ist.

Ein weiterer Gegenstand der vorliegenden Erfindung ist eine pharmazeutische Zusammensetzung, die als Wirkstoff mindestens ein in einen Träger eingebautes Chalkogen-enthaltendes, insbesondere ein Schwefel-enthaltendes, Aminosäureanalogon von Tryptophan umfasst, wobei der Träger ein Protein, ein Peptid oder eine peptidische Leitstruktur ist.

Vorzugsweise ist das in einen Träger eingebaute Analogon [3,2]Tpa oder [2,3]Tpa.

Vorzugsweise ist der Träger ein Säugerpeptid oder ein Säugerprotein, es können jedoch selbstverständlich auch Peptide oder Proteine aus anderen eukaryontischen Organismen oder aus Prokaryonten sowie rekombinante Peptide oder Proteine mit einer veränderten Aminosäure-Sequenz eingesetzt werden. In vielen Fällen wird vorzugsweise ein Träger verwendet, welcher sich in seiner Aminosäure-Sequenz von dem entsprechenden endogenen natürlichen Peptid oder Protein nur durch den Austausch einer kanonischen Aminosäure durch das Chalkogen-enthaltende Analogon von Tryptophan unterscheidet. Besonders bevorzugt ist das Chalkogen-enthaltende Analogon von Tryptophan in ein rekombinantes Säugerprotein eingebaut, welches eine Molekülmasse von ≥ 10 kDa aufweist.

Des Weiteren ist es bevorzugt, dass der ein Aminosäure-Analogon enthaltende Träger ein Peptid oder ein Protein ist, das eine mit einem entsprechenden Wildtyp-Protein, das heißt einem dieselbe Aminosäure-Sequenz, aber anstelle des Analogons die entsprechende kanonische Aminosäure aufweisenden Protein, weitgehend oder sogar vollständig eine identische dreidimensionale Struktur aufweist. Die Übereinstimmung der dreidimensionalen Strukturen von Analogon-modifiziertem Polypeptid und nicht-modifiziertem Polypeptid werden vorzugsweise durch Kristallstrukturanalyse, aber auch durch NMR-Spektroskopie bestimmt.

Voraussetzung für die Übereinstimmung der dreidimensionalen Strukturen der Träger-Proteine ist, dass das nicht-kanonische Aminosäureanalogon von Tryptophan ein Bioisoster von Tryptophan ist. Bioisostere sind isostere Moleküle, die nahezu gleiche Formen und Volumina, annähernd die selbe Verteilung an Elektronen aufweisen und vorzugsweise ähnliche oder antagonistische Eigenschaften in biologischen Systemen besitzen.

Der Versuch zur Herstellung von Indol-Isosteren, bei denen die Iminogruppe der Indolgruppe mit anderen Heteroatomen wie Schwefel oder Sauerstoff ersetzt wird, führte bislang zu Verbindungen, die die biologischen Eigenschaften verglichen mit jenen der Indol-Verbindung nicht vorhersagbar beibehielten. Dies liegt erstens daran, dass diese planaren Systeme in der enzymatischen Kondensationsreaktion mit Serin durch Tryptophan-Synthase nicht aktiviert waren. Zweitens, selbst wenn die entsprechenden Tryptophan-Analoga synthetisiert würden, würden sie wahrscheinlich nicht als Substrate für die Aktivierung von Tryptophanyl-tRNA-Synthetase in der Proteintranslation wirken. Schließlich waren selbst Änderungen an den Nachbarpositionen des protonierten Stickstoffatoms von Indol störend, da die Einführung eines zusätzlichen Stickstoffatoms in die Indolposition 2 zu 2-Aza-Tryptophan führt, das nicht als Substrat für die Proteinsynthese erkannt wird.

Dagegen führt die Einführung eines Stickstoffatoms in Position 7 des Indols zu 7-Aza-Tryptophan, das durch die zelluläre Tryptophanyl-tRNA-Synthetase erkannt wird und in Proteine eingebaut wird. Diese Erkenntnisse zeigen die universelle biologische Bedeutung der Indol-Imino-Funktion in Tryptophan, weshalb der Indol-Benzolring als Ziel für chemische Transformationen, die zu biologisch interessierenden isosteren Verbindungen führen, bevorzugt ist.

Die biomedizinischen Potentiale von aromatischen Systemen, die aus einem Pyrrolkern kondensiert an ein Thiophen bestehen, sind aufgrund ihrer Ähnlichkeiten mit Indolen seit längerem bekannt (Gronowitz et al., Acta. Chem. Scand. 1976, B30, 391 - 395). Ferner ist es bekannt, dass Thieno[3,2-b]-Pyrrol und Thieno[2,3-b]-Pyrrol bioisostere Analoga des Halluzinogens und Serotonin-Agonisten N,N'-Dimethyltryptamin sind (Blair et al., J. Med. Chem. 1999, 42, 1106 - 1111). Rekombinante Proteine, die pharmazeutisch aktive Aminosäuren wie die vorstehend beschriebenen Thienopyrrolyl-L-alanine enthalten, könnten aufgrund ihrer potentiellen Fähigkeit zum selektiven Transport und Targeting im menschlichen Körper als spezifische Shuttles oder sogar als "magic bullets" wirken (Minks et al., 2000, s.o.).

Die Auswahl der Trägerpeptide oder -proteine für das pharmakologisch wirksame Chalkogen-enthaltende Analogon von Tryptophan erfolgt günstigerweise entsprechend dem jeweiligen Einsatzzweck. Um eine möglichst zielgerichtete Verabreichung des Wirkstoffs zu erreichen, ist in vielen Fällen die Verwendung eines gewebespezifischen Peptids oder Proteins sinnvoll. Die Bezeichnung "gewebespezifisch" bedeutet in dem Zusammenhang der vorliegenden Erfindung, dass der Träger derart ausgewählt wird, dass er vorzugsweise durch gewebespezifische Mechanismen, beispielsweise eine Träger-vermittelte Aufnahme oder eine Rezeptor-vermittelte absorptive Endocytose oder Transcytose selektiv zu seinem beabsichtigten Wirkort gelangt. Dort kann der Träger in die gewünschte Zielzelle aufgenommen werden und innerhalb dieser Zelle physiologisch in einzelne Aminosäuren abgebaut werden, wobei das Chalkogen-enthaltende Analogon von Tryptophan als pharmakologischer Wirkstoff freigesetzt wird. Auf diese Weise ist es möglich, durch Auswahl geeigneter Träger den Wirkstoff gewebespezifisch an jeden gewünschten Ort des Körpers zu transportieren. Durch spezifische Rezeptor-vermittelte Endocytose und/oder adsorptive Endocytose kann beispielsweise sogar die Blut-Hirnschranke überwunden werden (Abbott und Romero, Molecular Medicine Today, März 1996, 106 - 113).

Die Art der Verabreichung der Trägerpeptide bzw. -proteine kann je nach Einsatzzweck oral, rektal, parenteral (intramuskulär, subkutan oder intravenös) oder durch Inhalation erfolgen. Die optimale Dosierung für den jeweiligen Einsatzzweck kann vom Fachmann durch einfache Versuche ohne weiteres ermittelt werden.

Bei einer besonders bevorzugten Ausführungsform werden die erfindungsgemäßen Proteine, Peptide und peptidischen Leitstrukturen, die mindestens ein Chalkogen-enthaltendes Analogon von Tryptophan als Wirkstoff enthalten, für die Herstellung von Halluzinogenen und/oder Serotonin-Agonisten verwendet.

Erfindungsgemäß werden somit neben pharmazeutischen Zusammensetzungen Verfahren bereitgestellt, die als einfache, kostengünstige, reproduzierbare und zuverlässige Methoden die Erkennung von Targetproteinen, -peptiden und peptidischen Leitstrukturen durch UV-Spektroskopie, Fluoreszenzspektroskopie und NMR-Spektroskopie sowie insbesondere die Strukturbestimmung durch Kristallographie beschleunigen.

Die nachfolgenden Beispiele dienen der Erläuterung der Erfindung, ohne diese einzuschränken.

### Beispiele

Alle Chemikalien außer den in Beispiel 1 beschriebenen Tryptophan-Analoga wurden, falls nicht anders angegeben, von Sigma Aldrich (Taufkirchen, Deutschland) bezogen. Die Fermentations-, Expressions- und Markierungsexperimente wurden in New Minimal Medium (NMM) unter Verwendung eines Tryptophan-auxotrophen *E.coli*-Stamms aus ATCC (Katalognr. 49980 (Genotyp: WP2 uvrA)) wie in Minks et al. (Biochemistry 1999, 38, 10649-10659) beschrieben durchgeführt. Die transformierten Wirtszellen wurden auf NMM angezogen (siehe Budisa et al., Eur. J. Biochem. 1995, 230, 788-796.; Budisa et al., 1997, siehe oben; Minks et al., 1999, siehe oben). Eine Lösung (20 mg/ml) des Tryptophan-Analogons wurde im Dunkeln bei -20°C aufbewahrt und ohne weitere Vorkehrungen routinemäßig für die Expression und Inkorporation bei einer Konzentration von 0,2 mM (50 mg/l) verwendet. Die Farbe der Kultur nach der Fermentation über Nacht mit dem Tryptophan-Aminosäure-Analogon war wie eine normale Tryptophan-enthaltende Kultur schwach gelblich, ohne dass ein Anzeichen des Abbaus dieser Tryptophan-Analoga sichtbar war.

### Beispiel 1: Synthese von Tryptophan-Analoga

### a) Methylazidoacetat

Methylbromacetat (306 g, 2 mol) wurde in 60 ml Ethanol und 180 ml Wasser gelöst. Natriumazid (143 g, 2,2 mol) wurde hinzugefügt und die resultierende Lösung wurde für vier Stunden unter Rückfluss gekocht. Danach wurde die leicht gelbliche Lösung destilliert, die organische Phase abgetrennt und über Na₂SO₄ aufbewahrt. Die Ausbeute ergab 101 g (44 %) (Houben-Weyl, Band XI/2, 353).

### b) Methyl-2-azido-3-(2'-thienyl)acrylat

Der große Vorteil dieser Methode, verglichen mit der bei Soth et al. (Can. J. Chem., 1978, 56, 1429) oder Welch und Phillips (Heterocyclic Comm., 1999, 5, 305) beschriebenen Methode ist, dass dabei weniger Nebenreaktionen auftreten sowie bessere Ausbeuten und bessere Reaktionsbedingungen vorliegen. Insbesondere ist die Verwendung eines geringeren Überschusses von Methylazidoacetat notwendig. Natrium (1,84 g) wurde in trockenem Methanol (55 ml) gelöst und die Lösung wurde auf -20°C abgekühlt. Eine Mischung von Thiophen-2-carbaldehyd (1,83 g, 16,3 mmol) und Methylazidoacetat (4,7 g, 40,8 mol) wurde tropfenweise zugegeben. Die Reaktionsmischung wurde bei 0°C gerührt, bis der gesamte Aldehyd verbraucht wurde (etwa 4 Stunden). Die Reaktionsmischung wurde in Eiswasser gegossen und mit Ether extrahiert (3 x 100 ml). Die vereinigten Etherextrakte wurden mit Wasser gewaschen, getrocknet, verdampft und durch Silica-Gelchromatographie gereinigt (2:1 n-Hexan/CH₂Cl₂). Die Ausbeute betrug 2,2 g (65 %) leicht gelbliche Kristalle (Knittel, Synthesis 1986, 186).

### c) 5-Carbomethoxythieno[3,2-b]pyrrol

Eine Lösung des Azids (2,9 g, 13,9 mmol) in Xylen (40 ml) wurde eine Stunde lang unter Rückfluss gekocht. Das Lösungsmittel wurde verdampft. Als Ausbeute wurden 2,5 g (99 %) rot-braune Kristalle erhalten.

### d) 5-Carboxythieno[3,2-b]pyrrol

Lithumhydroxid (476 mg, 20 mmol) wurden zu 1,8 g (10 mmol) 5-Carbomethoxythieno[3,2-b]pyrrol in Ethanol zugegeben. Die Mischung wurden bei 40°C bis zum Ende der Veresterung gerührt (etwa 10 Stunden). Das Lösungsmittel wurde verdampft, und der Rest in Ether gelöst und mit 5 % KHSO₄ gewaschen. Die organische Phase wurde abgetrennt, über Na₂SO₄ getrocknet und erneut verdampft. Als Ausbeute wurden 1,42 g (85 %) 5-Carboxythieno[3,2-b]pyrrol als braune Kristalle erhalten (Wensbo et al., Tetrahedron, 1995, 51, 10323).

### e) Thieno[3,2-b]pyrrol

Kupfer (93 mg, 1,47 mmol) wurde zu einer Lösung von 5-Carboxythieno[3,2-b]pyrrol (366 mg, 2,19 mmol) und Chinolin (2,5 ml) zugegeben. Die Reaktionsmischung wurde eine Stunde lang unter Argon bei 220 °C erwärmt, nach Abkühlung in 50 ml Ether verdünnt und die Feststoffe wurden abgefiltert. Das Filtrat wurde mit 3N HCl und NaHCO₃ extrahiert und über Na₂SO₄ getrocknet. Die Verdampfung des Lösungsmittels und Chromatographie (3:2 CH₂Cl₂/n-Hexan) des Rests ergab 156 mg (58 %) Thieno[3,2-b]pyrrol als braunes Öl (Palicka-Chodowska et al., Acta Chem. Scand., 1970, 24, 3353).

### f) β-Thieno[3,2-b]pyrrolyl-L-alanin

7 mg des gereinigten α₂β₂-Tryptophansynthase-Komplexes wurden zu einer Suspension von Thieno[3,2-b]pyrrol (156 mg, 1,27 mmol), L-Serin (200 mg, 1,9 mmol) und Pyridoxal-5'phosphat (0,5 mg, 1,9 µmol) in 60 ml 0,1 M Kaliumphosphatpuffer (pH 7,8) zugegeben. Die Reaktion wurde vorsichtig unter Argon bei 37°C geschüttelt, bis die gesamte Menge an Thieno[3,2-b]pyrrol gemäß HPLC verbraucht wurde (nach etwa 12 Stunden). Nach dieser Zeit wurde die Reaktionsmischung durch eine Centiprep-10 (Amicon) gefiltert, um den Tryptophan-Synthasekomplex zu entfernen, aufkonzentriert und die Ausbeute wurde mittels des UV-Profils (UV (H₂O), λₘₐₓ = 261 nm (log ε = 3,75) auf 230 mg (86 %) β-Thieno[3,2-b]pyrrolyl-L-alanin berechnet (Phillips et al., Bioorg. Med. Chem. Lett., 1995, 5, 1133).

### Beispiel 2: Fermentation, Expression und Reinigung von Barstar und Annexin V

Barstar, ein kleiner bakterieller Ribonuklease-Inhibitor, ist ein geeignetes Modellprotein für Untersuchungen der Inkorporation von nicht-kanonischen Tryptophan-Analoga, da es die folgenden Kriterien erfüllt: a) Sowohl Hochauflösungs-Kristall- und Lösungsstrukturen sind bekannt (Martin et al., FEBS Lett. 1999, 452, 128-132). b) Ferner unterliegt Barstar sowohl bei thermischer als auch bei chemischer Denaturierung einer vollständig reversiblen Entfaltung. c) Schließlich enthält Barstar drei Tryptophan-Reste an den Positionen 38, 44 und 53.

In den folgenden Beispielen wurden die Tpa-Substitutionsexperimente unter Verwendung der Pseudo-Wildtypmutante b¹* (Barstar C40A/C82A/P27A/W38F) durchgeführt, das in Form von inclusion bodies exprimiert wird und während des Reinigungsvorgangs in die native Form zurückgefaltet wird (Golbik et al., prot. sci. 1999, 8, 1505 - 1514). Die Mutante b¹* enthält lediglich zwei Tryptophan-Reste, nämlich W44, das teilweise und W53, das vollständig in das hydrophobe Protein-Core eingeschlossen ist (siehe Abbildung 1). W53 ist wesentlich für die Konformation des Proteins, da er durch keine der 20 kanonischen Aminosäuren durch Routine-DNA-Mutagenese ersetzt werden kann (Nath und Udgaonkar, Biochemistry 1997, 36, 8602 - 8610).

Ein zweites Modellprotein, das die vorstehend genannten Bedingungen erfüllt, ist das rekombinante humane Annexin V (AxV), das in löslicher Form unter der Kontrolle des T5-Promoter/Polymerase-Systems exprimiert wird. AxV bindet in Kalzium-abhängiger Weise an saure Phospolipid-Membrankopfgruppen (Huber et al., EMBO J. 1990, 9, 3867 - 3874; Berendes et al., Science 1993, 262, 427 - 430 ; Liemann und Huber, Cell Mol. Life Sci. 1997, 53, 516 - 521). AxV enthält lediglich einen, aber wesentlichen Tryptophan-Rest (W187), der in kristallinem Zustand zwei molekulare Konformationen besitzen kann: Eine, in der W187 in die hydrophobe Nische der Domäne III eingeschlossen ist (siehe Abbildung 1), und eine andere, in der W187 vollständig dem Bulk-Lösungsmittel ausgesetzt ist (Berendes et al., 1993, s.o.; Concha et al., Science 1993, 261, 1321 - 1324).

In den folgenden Beispielen wurden W187 von Annexin V sowie die zwei Tryptophan-Reste von b* durch [3,2]Tpa bzw. [2,3]Tpa unter Verwendung des Tryptophan-auxotrophen *E. coli*-Stamms ATCC 49980 und eines Expressionsystem auf T5-Basis mit der SPI-Methode wie in Abbildung 1 dargestellt ersetzt.

Methionin-auxotrophe *E. coli*-Stämme wachsen in Gegenwart von Selenomethionin als einziger Methionin-Quelle in Minimalmedium aufgrund dessen moderater Toxizität für die Zellen (Budisa et al., 1995, s.o.). Dies gilt jedoch nicht für die Thia-Analoga von Tryptophan (siehe Abbildung 3A). Das Wachstum des E. coli-Stamms ATCC 49980 wurde mit Beginn der Fermentierung im definierten Minimalmedium, welches 0,015 mM (3,1 µg/ml) einer jeden Thia-Aminosäure als einziger Quelle für Tryptophan enthält, stark inhibiert. Als die β-(Thienopyrrolyl)-Alanine zusammen mit Tryptophan in einem Verhältnis von 1:1 der Fermentation zugegeben wurden, erlaubte diese Mischung überraschenderweise das Wachstum der Zellen bis zu einer stationären Phase, d.h. das Wachstum wurde offensichtlich selbst dann unerstützt, nachdem der Tryptophan-Vorrat erschöpft war.

Diese Ergebnisse zeigen deutlich, dass die Thieno-Analoga von Tryptophan in einer Mischung mit Tryptophan als Vorrat für das zelluläre Wachstum nicht nur von den auxotrophen Zellen toleriert werden, sondern in gewissem Maße als Substrate für das zelluläre Wachstum verwendet werden. Somit ist die Toxizität dieser Substanzen verglichen mit Fluoro-Tryptophan-Analoga relativ gering, aber im Vergleich zur Toxizität von Selenomethionin immer noch relativ stark. Ferner erwiesen sich die freien Aminosäuren [3,2]Tpa und [2,3]Tpa in allen Fermentationsexperimenten stabil, da trotz dem Aussetzen an Luft und Licht für einige Tage kein Abbau dieser Aminosäuren beobachtet wurde.

Der *E*. *coli*-Wirt ATCC49980 wurde routinemäßig mit zwei Plasmiden co-transformiert: dem Ampicillin-resistenten Plasmid pQE-30-PP4, umfassend das Annexin V-Gen unter der Kontrolle des T5-Promotors und dem Kanamycin-resistenten Plasmid pREP4, enthaltend ein Repressorgen lacI^{q}.

Das selbe System wurde für die Expression der Barstar-Mutante b²* (Barstar C40A/C82A/P27A) für die Sep-Substitutionsexperimente verwendet: das EcoRI-HindIII-Fragment pKK223-3-Plasmid, enthaltend die Ribosom-Bindungsstelle und die b²*-Sequenz wurden in einen pQE-30-Vektor (Qiagen, Hilden, Deutschland) insertiert und zusammen mit pREP4 in den Expressionswirt transformiert.

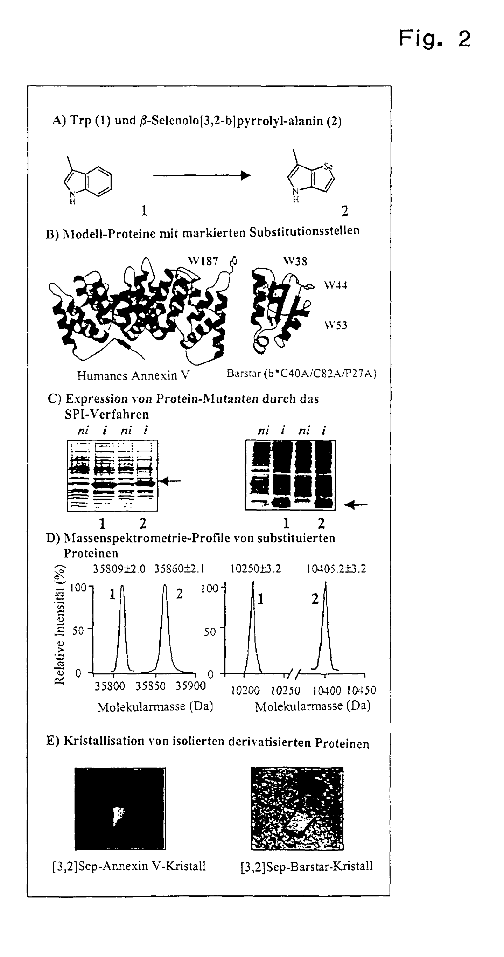

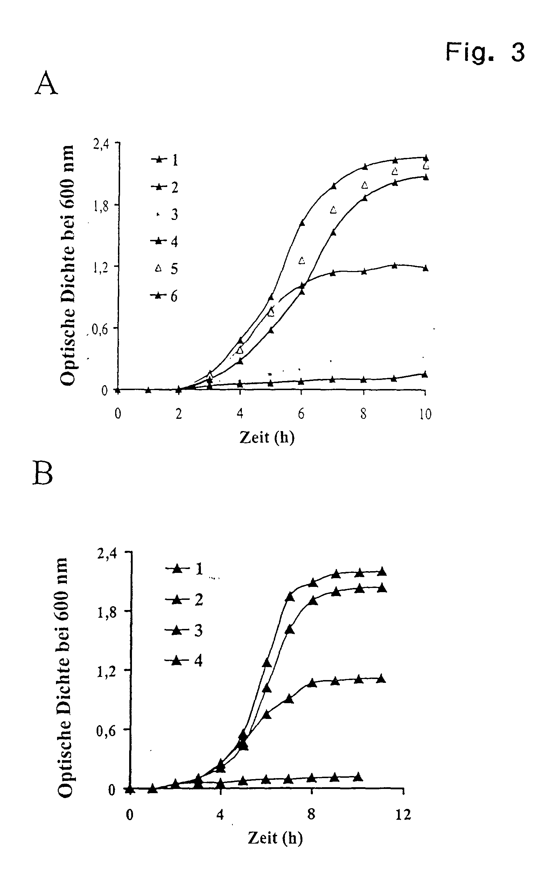

Der *E. coli*-Stamm ATCC 49980 zeigte in Minimalmedium mit 0,3 mM Tryptophan typische Wachstumskurven. Die Inkorporationsexperimente wurden unter Verwendung von in NMM angezogenen Kulturen in Gegenwart von 100 mg/l Ampicillin und 70 mg/l Kanamycin und 0,015 mM Tryptophan als optimale limitierende Konzentration des nativen Substrats bei 30°C (siehe Abbildung 2) durchgeführt. Dieses Medium liefert genügend "gesunde" Zellen vor dem Verschwinden von Tryptophan als nativem Substrat in der Mitte der logarithmischen Phase des Wachstums (OD₆₀₀ etwa 0,7-0,9), die einen optimalen Punkt zur Zugabe des nicht-kanonischen Aminosäure-Substrats darstellt. Die Expression des Targetproteins wird durch Zugabe von 1 mM IPTG angeschaltet. Die Fermentationsdauer nach der Induktion der Proteinsynthese betrug 4 Stunden oder über Nacht bei 30°C für Annexin V und bei 26°C für b*C40A/C82A/P27A.

Unter diesen Bedingungen wurde eine optimale Produktion der markierten Proteine in Ausbeuten erreicht, die vergleichbar mit jenen der jeweiligen Wildtypproteins sind (10 - 30 mg/l im Fall von Annexin V und 50 - 100 mg/l im Fall von Barstar b*).

Die Reinigungsverfahren für die markierten Derivate beider Proteine waren identisch zu jenen, die für die Wildtyp-Formen beschrieben wurden (Budisa et al., 1995; siehe oben). Die Reinheit der Proteine wurde durch SDS/PAGE (Silberanfärbung), HPLC und Elektronenspray-Massenspektrometrie bestimmt.

### Beispiel 3: Analytische und spektroskopische Verfahren

### a) Fluoreszenz

In Routine-Bioexpressionsprotokollen basierend auf dem SPI-Verfahren wurde eine nahezu quantitative Inkorporation der beiden β-Thienopyrrolyl-Alanine in AxV und b¹* erreicht (siehe Abbildung 1). Um den Einbau dieser Aminosäuren zu bewerten, wurden Fluoreszenzprofile der Proteinmutanten aufgenommen, da der Ersatz des Benzolrings der Indolgruppe mit Thiophen zu einem effizienten Fluoreszenz-Quenching führt (siehe Abbildung 4). Somit wurde die Abwesenheit des charakteristischen Tryptophan-Emissionsfluoreszenz-Profils als qualitative analytisches Kriterium zur Aufzeichnung der erfolgreichen Markierung verwendet.

Die Fluoreszenzspektren wurden auf einem Perkin-Elmer-Spektrometer, ausgestattet mit digitaler Software (LS50B), aufgenommen. Die Proteinproben (1,0 µM Annexin, 0,5 µM Barstar-Mutante) wurden in Phosphat-Salzlösung oder in 50 mM Natriumdihydrogenphosphat (pH 8,0) hergestellt. Die Proteinproben wurden bei 280 nm (Spalt 2,5 nm) angeregt und die Emissionsspektren wurden im Bereich von 310 bis 450 nm aufgenommen.

### Massenspektrometrie

Der quantitative Ersatz der nativen Tryptophan-Reste durch nicht-kanonische Thieno-Pyrrol-Alanine wurde routinemäßig durch Elektronenspray-Massenspektrometrie (ESI-MS), wie bereits von Budisa et al., 1995 (siehe oben) beschrieben, bestätigt.

Die molekulare Massendifferenz zwischen Tryptophan und beiden Thia-Varianten ist mit 6 Da ausreichend groß, um experimentell bestimmt zu werden. Die in Abbildung 1 gezeigten Daten bestätigen neben dem erfolgreichen Einbau, dass die einmal in die Proteine eingebauten Thia-Analoga stabil sind.

### c) Analyse der UV/VIS-Absorptionsprofile

Die UV-Absorptionsspektren von Proteinen und Aminosäuren in Pufferlösungen wurden routinemäßig mit einem Perkin-Elmer Lambda 17 UV/VIS-Spektrophotometer aufgenommen. Die Extinktionskoeffizienten für native und substituierte Proteine wurden aus der Aminosäurezusammensetzung gemäß dem von Mach et al. (Anal. Biochem. 1992, 200, 74-80) beschriebenen Verfahrens bestimmt: Die Extinktionskoeffizienten für native und substituierte Proteine (siehe Tabelle 2) wurden aus der quantitativen Aminosäureanalyse der Säurehydrolysate (6 M HCl, 24 h, 110°C) bestimmt. Die Extinktionskoeffizienten für die Aminosäuren in der zwitterionischen Form (siehe Tabelle 2) wurden in 50 mM Natriumdihydrogenphosphat, pH 8,0 bei Konzentrationen von 100 µM für L-Trp, 82 µM für [3,2]Tpa und von 54 µM [2,3]Tpa bei 20°C bestimmt.

Der Extinktionskoeffizient des Absorptionsmaximums für [3,2]Sep als freie Substanz in neutraler Pufferlösung ist der Literatur entnommen (Welch und Phillips, Bioorg. Med. Chem. Lett. 1999, 9, 637-640) und beträgt 12882 M⁻¹cm⁻¹(λₘₐₓ=267 nm).

Die UV-Spektra der Proteinmutanten werden durch den Einbau der zwei β-Thienopyrrolylalanine an den Tryptophan-Positionen beeinflusst. Die UV-Spektra der AxV-Mutanten wurden nicht wesentlich verändert, da der Beitrag der Tyrosin-Reste zur gesamten Absorption dominant ist (siehe Abbildung 5 I C). Dagegen sind die UV-spektroskopischen Eigenschaften der β-Thienopyrrolyl-Alanine in Spektren der Barstar-Mutanten mit einer Blauverschiebung des Absorptionsmaximums vollständig wiedergegeben (siehe Tabelle 2 und Abbildung 5 I B). Derartige Blauverschiebungen entstehen durch die hydrophobe Umgebung der teilweise (Position 38) und vollständig eingeschlossenen aromatischen β-substituierten Alanine (Position 53).

**Tabelle 2**

| Rest | Freie Aminosäure | | Annexin V | | Barstar b* | |
|---|---|---|---|---|---|---|
| | ε_{M}^{b} | λ_{*max*}^{*a*} | ε_{M} | *λ*_{*max*} | ε_{M} | *λ*_{*max*} |
| L-Trp | 5579 ± 257 | 280 | 21500 ± 931 | 277 | 14920 ± 531 | 280 |
| [3,2]Tpa | 5632 ± 373 | 260 | 21500 ± 791 | 277 | 13060 ± 197 | 261 |
| [2,3]Tpa | 4897 ± 169 | 241 | 22800 ± 287 | 277 | 10712 ± 331 | 250 |

| | | | | | | |
|---|---|---|---|---|---|---|
| ^{a} angegeben in *nm*; | | | | | | |
| ^{b} angegeben in *M*^{*-1*}*cm*^{*-1*}, ^{c} in 50 mM Na-Dihydrogenphosphat pH 8.0; ^{d} in 10 mM TrisCl pH 8.0 | | | | | | |

### d) Circulardichroismus

Die fernen UV- und nahen UV-CD-Spektren wurden bei 20°C auf einem JASCO J-715-Spektrometer in einer in dem JASCO-Hardware Manual P/N: 0302-0265A (1995) beschriebenen Konfiguration aufgenommen. Die Spektren zur Bestimmung der Sekundärstruktur wurden mit Proteinkonzentrationen von 0,08 mg/ml Annexin V in PBS und 0,2 mg/ml b* in 50 mM Natriumdihydrogenphosphat, pH 8,0 durchgeführt. Die Spektren wurden in Hellma 110-QS Quarzzellen mit 0,1 cm optischer Weglänge gemessen. Ein ausreichendes Signal-zu-Rausch-Verhältnis wurde durch Aufnahme von vier Durchläufen der fernen UV-CD-Spektren erreicht.

Die nahen UV-CD-Spektren wurden bei Proteinkonzentrationen von 0,5 mg/ml in PBS (Annexin V) oder in 50 mM Natriumphosphat, pH 8,0 (b*) in Hellma 110-QS-Zellen mit 1,0 cm optischer Weglänge aufgenommen. Pro Spektrum wurden zehn Scans durchgeführt und die Rohdaten wurden unter Verwendung des Verfahrens in der JASCO-Software (Softwaremanual P/N: 0302/0266A, 1995) prozessiert.

Die fernen UV-CD-Spektren von Annexin V und dessen Mutanten zeigen das typische Muster von α-helikalen Proteinen mit zwei charakteristischen Minima bei 222 nm und 208 nm bei ähnlicher Intensität (siehe Abbildung 5 III A Insert). Das Signalverhältnis zwischen diesen beiden Minima ([θ]_{R})₂₂₂/([θ]_{R})₂₀₈ beträgt 0,96 sowohl für das Wildtyp-Protein als auch für die [2,3]Tpa-Mutante und ist nur geringfügig vermindert für die [3,2]Tpa-Mutante (0,95). Auf ähnliche Weise zeigen die b¹*-Mutante und dessen [2,3]Tpa-b¹*-Analogon nahezu identische Spektren und Signalverhältnisse (∼0,90), während dieses Verhältnis für [3,2]Tpa-b* deutlich erhöht ist (0,97) (siehe Abbildung 5 III A).

Das nahe UV-CD-Spektrum von Wildtyp-Annexin V zeigt den Beitrag der Phenylalanin-Reste mit der scharfen feinen Struktur (255 - 270 nm) der Tyrosin-Reste mit dem Maximum zwischen 275 und 282 nm und der Tryptophan-Reste mit dem Maximum um 290 und 305 nm (Minks et al., 1999, s.o.). Der Ersatz dieser Tryptophan-Reste durch die zwei β-Thienopyrrolyl-Alanine führt zu deutlichen spektralen Veränderungen in der aromatischen Region, die hauptsächlich durch die intrinsischen dichroistischen Eigenschaften dieser Tryptophan-Varianten entstehen. Ein Vergleich der nahen UV-CD-Spektren von Tryptophan und den β-(Thienopyrrolyl)-Alaninen in Form von freien Aminosäuren zeigte, dass die Spektren von Tryptophan und [2,3]Tpa mehr oder weniger ähnlich sind, während das Spektrum von [3,2]Tpa eine starke positive Bande zwischen 280 und 250 nm zeigte. Dies wird vollständig in den Spektren der beiden Mutanten-Proteine und insbesondere von b¹* wiedergegeben, bei dem der Beitrag der Tryptophan-Reste zum gesamten Proteinspektrum dominant ist (siehe Abbildung 5 III C).

Im nahen UV sind die CD-Spektren der [2,3]Tpa-enthaltenden Proteine ähnlich zu denen der nativen Proteine (siehe Abbildung 5 III) und zeigen lediglich geringe Unterschieden in der dichroistischen Intensität der positiven Banden. Dieser Effekt ist stärker für Wildtyp-Annexin V als für [2,3]Tpa-Annexin V (siehe Abbildung 5 III B), aber schwächer für das b¹*-Protein verglichen mit dessen [2,3]Tpa-Mutante (siehe Abbildung 5 III C).

Dagegen führt der Ersatz von Tryptophan-Resten mit [3,2]Tpa in beiden Proteinen zu drastischen Veränderungen der nahen UV-CD-Spektren (siehe Abbildung 5 III B und C). Im nahen UV-CD-Spektrum von [3,2]Tpa-AxV dominiert diese Änderung die spektrale Region zwischen 250 und 268 nm, während zwischen 270 und 290 nm die Intensitäten verglichen mit dem Spektrum des Wildtyp-Proteins nur unwesentlich verringert werden (siehe Abbildung 5 III B). Ein ähnlicher, sogar noch stärkerer Effekt wird für [3,2]Tpa-b¹* beobachtet (siehe Abbildung 5 III C), wobei die Änderungen aus einer starken lokalen Veränderung der räumlichen Anordnung von benachbarten aromatischen Resten oder aus der intrinsischen spektralen Natur von [3,2]Tpa als Chromophor sowie aus der Kombination dieser beiden Effekte resultieren könnten.

Ein Vergleich der fernen UV-CD-Spektren aller Annexin V-Proteine zeigt, dass die gesamten dichroistischen Muster nahezu identisch sind (siehe Abbildung 5 III A), während für [3,2]Tpa-b* das Spektrum verglichen mit der nativen Proteinvariante um 208 nm geringfügig unterschiedlich ist. Dies könnte geringfügige Änderungen der Sekundärstruktur dieses Mutanten anzeigen. Jedoch können drastische Veränderungen in der aromatischen Region das ferne UV-CD-Spektrum beeinflussen, ohne Änderungen in der Sekundärstruktur herbeizuführen, wie es bereits in anderen Systemen beobachtet wurde (Woody und Dunker, Aromatic and cystine side-chain circular dichroism in proteins, Hrsg.: G. D. Fasman, Circular dichroism and the conformational analysis of biomolecules, Plenum press, New York, 1996).

### Beispiel 4: Kristallisation und Röntgenstrukturanalyse

Für die Kristallisation von [3,2]Sep-Annexin V wurde die Methode des "hanging drop" (hängender Tropfen) bei Raumtemperatur mittels Dampfdiffusion gegen 2,0 M Ammoniumsulfat, gepuffert mit 0,1 M Tris/HCl (pH 8,5) angewendet. Das Protein wurde gegen einen 10 mM Tris/HCl-Puffer (pH 8,5) dialysiert und auf 10 bis 20 mg/ml aufkonzentriert. Zu 10 µl der Proteinlösung wurden 1 µl 20 mM CaCl₂-Lösung und 1,5 µl von 2,0 M gepuffertem Ammoniumsulfatniederschlag mit Agarose zugefügt, um eine Endkonzentration von 0,1 % oder 0,05 % gemäß dem von Robert et al. (Crystallisation in gels and related methods. In: *Crystallisation of Nucleic Acids and Proteins - A Practical Approach* (Hrsg.: A. Ducruix und R. Giege) 1991, Iril Press at Oxford University Press. Oxford, New York, Tokio) beschriebenen Verfahren zu erreichen.

Die Herstellung der Tropfen in Agarose wurde in drei Schritten wie folgt erreicht: Zunächst wurde eine 2 %ige Agarose-Lösung in destilliertem Wasser hergestellt. Dann wurde die geschmolzene Agarose mit Reservoir-Puffer gemischt. Beispielsweise ist es zur Herstellung von 10 ml des Niederschlags in 0,2 % Agarose erforderlich, 3 ml Reservoir-Puffer, 2 ml 20 mM Ca²⁺, 4 ml Wasser und 1 ml 2% Agarose zu mischen. Diese Mischung wurde in gleiche Teile aufgeteilt und bei 42°C aufbewahrt. Schließlich wurden 10 µl von [3,2]Sep-Annexin V (10 bis 20 mg/ml) mit 10 µl des Niederschlags in 0,2 % Agarose bei 42°C gemischt und die resultierenden hängenden Tropfen mit einer Endkonzentration von Agarose von 0,1 % wurden abkühlen gelassen. Die Kristalle (siehe Abbildung 1) wuchsen innerhalb von 5 bis 9 Tagen und wurden in 3 M Ammoniumsulphat, 0,1 M Tris/HCl (pH 8,5) und 1 mM CaCl₂ geerntet.

Die Kristallisation der Barstar-Mutante b²* (Barstar C40A/C82A/P27A) wurde unter Verwendung der "sitting drop"-Methode bei Raumtemperatur (18-20°C) in "Cryschem"-Platten von Hampton Research Ltd. (Laguna Niguel, USA) durchgeführt. Die Proteinkristalle wuchsen innerhalb von 3 bis 5 Tagen aus der Proteinlösung (2,5 mg/ml) in 0,9 bis 1,1 M Natriumcitrat, gepuffert mit 50 mM Tris-Cl-Puffer, pH 8,0 (siehe Abbildung 1). Diese Kristallisationsbedingungen sind ähnlich zu jenen des nativen Cystein-freien Barstars und seiner verschiedenen Mutanten (Martin et al., FEBS Lett. 1999, 452, 128-132). Da die b²*-Kristalle nicht stabil genug waren, um einen vollständigen Diffraktionsdatensatz bei Raumtemperatur zu erhalten, wurden sie in ein kapillarfreies System in einem Kristallisationspuffer mit 20 % Glycerol für den Cryo-Schutz aufgebracht.

Die Kristallgitterkonstanten, Raumgruppen und die Diffraktionsqualität sind in Tabelle 3 dargestellt. Die Diffraktionsdaten wurden von einem Einkristall des jeweiligen Proteins auf einem X-Ray-Image-Plate-System (MarResearch, Hamburg, Deutschland) unter Verwendung von CuK_{α}-Strahlung von einem Rigaku-Drehanoden-Röntgengenerator, betrieben bei 5,4 kW mit einer Auflösung von 2,4 Å ([3,2]Sep-b²*) bzw. 2,3 Å ([3,2]Sep-Annexin V) erhalten. Bei Annexin V wurde das Experiment bei 18 °C durchgeführt, und bei den b²*-Kristallen wurde ein in einem Loop montierter Proteinkristall (MarResearch, Hamburg, Deutschland) unter Einsatz eines Cryo-Systems gemessen und während des Diffraktionsexperiments in flüssigem Stickstoff bei 100 K gehalten.

Die Reflektionen wurden mit dem Programm MOSFLM 551 integriert, skaliert und unter Verwendung von SALEPACK des CCP4-Packages (Collaborative Computational Project No. 4) reduziert. Die Statistik ist in Tabelle 3 angegeben. Die kristallographischen Berechnungen wurden hauptsächlich mit MAIN und X-PLOR durchgeführt. Die Atomsubstitutionen wurden durch die Differenz-Fourier-Methode bestimmt. Die Differenz-Karten wurden auf einem Computergraphikdisplay untersucht und unter Verwendung der Programme MAIN und MOLEMAN ausgedruckt.

**Tabelle 3**

| **[3,2]Sep-Annexin V** | | **[3,2]Sep- b**^{**2**}*** (Barstar C40A/C82A/P27A)** | |
|---|---|---|---|
| Raumgruppe | R3 | Raumgruppe | R3 |
| Zellkonstanten | a = b = 157,31 c = 35,91 | Zellkonstanten | a = b = 116,50 c = 36,51 |
| Limitierende Auflösung (Å) | 2,3 | Limitierende Auflösung (Å) | 2,4 |
| Eindeutige Reflexionen | 18156 | Eindeutige Reflexionen | 7134 |
| Completeness | 92,8 % | Completeness | 98,2 % |
| R_{merge} ^{*} | 11,7 % | R_{merge} ^{*} | 7,9% |
| Moleküle pro asymmetrische Einheit | 1 | Moleküle pro asymmetrische Einheit | 2 |
| Refinement: | | Refinement: | |
| Auflösungsbereich (Ä) | 500 - 2,3 | Auflösungsbereich (Å) | 29,7 - 2,4 |
| Kristallographischer R-Faktor^{†} | 23,7 % | Kristallographischer R-Faktortz^{†} | 22,8 % |
| Freier R-Faktor^{††} | 27,7 % | Freier R-Faktor^{††} | 29,5% |
| Rms-Abweichungen: | | Rms-Abweichungen: | |
| Bindungslängen | 0,010 Å | Bindungslängen | 0,008 Å |
| Bindungswinkel | 1,34 ° | Bindungswinkel | 1,25 ° |
| Anzahl der Nicht-H-Atome | 2475 | Anzahl der Nicht-H-Atome | 1438 |
| Lösungsmittelmoleküle | 137 | Lösungsmittelmoleküle | 73 |

| | | | |
|---|---|---|---|
| *R_{merge} = σ[I(h)ᵢ-〈I(h)〉]/σ〈I(h)〉; I(h)ᵢ ist die beobachtete Intensität der i-ten Messung der Reflexion h, und I(h) die mittlere Intensität der Reflexion h, berechnet nach dem Laden und Skalieren | | | |
| † R_{Faktor} = σ[\|F_{obs}\| - \|F_{calc}\|]/\|F_{obs}\| × 100 | | | |
| †† R_{frei} wurde berechnet, indem bei dem Refinement und der R-Faktor-Berechnung statistisch 10 % (bei Barstar 5 %) der beobachteten Reflexionen weggelassen wurden. | | | |

### Beispiel 5: Biologische Assays

Von Annexin V und den Barstar-Mutanten b¹* und b²* wurde jeweils die native und die substituierte Form quantitativ auf ihre biologische Aktivität getestet. Die Barstar-Mutanten und deren Varianten wurden auf ihre inhibitorische Aktivität gegen Barnase getestet (Golbick et al., Prot. Sci. 1999, 8, 1505-1514), während Annexin V und dessen Varianten auf deren Fähigkeit zur effektiven Bindung an Phospholipid-enthaltende Membrane in der Gegenwart von hohen Kalziumkonzentrationen getestet wurden (Budisa et al., Eur. J. Biochem. 1998, 53, 1-9).

### Abbildungen

Abbildung 1: Schematische Darstellung der experimentellen Schritte für die effiziente *in* vivo-Inkorporation von Tpa in Gegenwart des Tryptophan-Codons in den Target-Genen der Modellproteine durch Verwendung des SPI-Verfahrens.
A) Strukturelle Darstellungen der Seitenketten der kanonischen Aminosäure Tryptophan **(1)** und seiner nicht-kanonischen Analoga [2,3]Tpa (β-3-Thieno[2,3-b]pyrrolyl-L-alanin) **(2)** und [3,2]Tpa (β-3-Thieno[3,2-b]pyrrolyl-L-alanin) **(3)**.
B) Bänderdarstellung der Modellproteine Annexin V (rechts) und Barstar b¹* (links). Die Positionen der jeweiligen Tryptophan-Reste sind angegeben.
C) Analyse der Expressionsprofile in Zelllysaten von *E.coli* ATCC49980 mit Annexin V (links) und b¹* (rechts) in definiertem Minimalmedium mit Tryptophan **(1)** und [2,3]Tpa **(2)** und [3,2]Tpa (**3**). ni steht für nicht-induzierte Zellen und i steht für induzierte Zellen in der Gegenwart von kanonischen und nicht-kanonischen Substraten. Die Pfeile zeigen die Position der überexprimierten substituierten Proteine in SDS-PAGE-Gelen (Comassie-Blue-Anfärbung, Bio-Rad).
D) Analytischer Nachweis der Substitutionen durch Elektronenspray-Massenspektrometrie von nativen Proteinen und den entsprechenden Mutanten. Zwei unterschiedliche Elektrospray-Massenspektrometriemessungen wurden auf der selben Massenskala überlagert (links: natives und [2,3]Tpa-Ax V; rechts: Wildtyp- und [3,2]Tpa-b¹*.

Abbildung 2: Schematische Darstellung der experimentellen Schritte für die effiziente *in vivo*-Inkorporation von [3,2]Sep in Gegenwart des Tryptophan-Codons UGG in den Target-Genen der Modellproteine durch Verwendung des SPI-Verfahrens.
A) Strukturelle Darstellungen der Seitenketten der kanonischen Aminosäure Tryptophan und seines nicht-kanonischen Analogons [3,2]Sep (β-3-Selenolo[3,2-b]pyrrolyl-L-alanin).
B) Bänderdarstellung der Modellproteine Annexin V (rechts) und Barstar b²* (links). Die Positionen der jeweiligen Tryptophan-Reste sind angegeben.
C) Analyse der Expressionsprofile in Zelllysaten von *E.coli* ATCC49980 mit Annexin V (links) und b²* (rechts) in definiertem Minimalmedium mit Tryptophan **(1)** und [3,2]Sep **(2)**. ni steht für nicht-induzierte Zellen und i steht für induzierte Zellen in der Gegenwart von kanonischen und nicht-kanonischen Substraten. Die Pfeile zeigen die Position der überexprimierten substituierten Proteine in SDS-PAGE-Gelen (Comassie-Blue-Anfärbung, Bio-Rad).
D) Analytischer Nachweis der Substitutionen durch Elektronenspray-Massenspektrometrie von nativen Proteinen und den entsprechenden Mutanten. Der Ersatz des einzigen Tryptophans 187 in Annexin V mit [3,2]Sep führt zu einem Anstieg von 50 Da in der Proteinmolekülmasse. Barstar b²* enthält drei Tryptophan-Reste, weshalb der Massenunterschied (150 Da) durch die Substitution größer ist. Tatsächlich ist die Übereinstimmung zwischen der erwarteten und der berechneten Molekülmasse gut (gefunden: 10405,2 ± 3,2 Da; berechnet: 10403 Da). In sämtlichen Massenspektren der Proteine mit Aminosäure-Analoga von Tryptophan wurden keine Signale von Wildtyp-Proteinspezies detektiert.
E) Photographien der Kristalle.

Abbildung 3:
A) Die Wachstumskurven des transformierten Tryptophan-auxotrophen *E. coli*-Stamms ATCC49980 bei 30°C in NMM-Medium mit unterschiedlichen L-Trp- und [2,3]Tpa- bzw. [3,2]Tpa-Konzentrationen.
   Die Zellen wurden mit zwei Plasmiden co-transformiert: Ampicillin-resistentes Plasmid pQIA-30 b* mit dem Gen für Barstar b* und das Kanamycin-resistente Plasmid pREP**4**, enthaltend ein Repressorgen lacI^{q} (Qiagen). Sechs verschiedene in NMM-Medium aufgezogene Kulturen wurden mit den folgenden Mengen an Tryptophan als nativem Substrat und den β-(Thienopyrrolyl)-Alaninen gefüttert: **1**: 0,03 mM L-Trp; **2**: 0,015 mM L-Trp; **3**: 0,015 mM [2,3]Tpa; **4**: 0,015 mM [3,2]Tpa; **5**: 0,015 mM [2,3]Tpa + 0,015 mM L-Trp; **6**: 0,015 mM [3,2]Tpa + 0,015 mM L-Trp.
   Die Mischungen von L-Trp mit [2,3]Tpa bzw. [3,2]Tpa (5, 6) ermöglichen den Zellen, die stationäre Phase zu erreichen, führen aber zu einer erniedrigten Gesamtwachstumsrate. Das Wachstum wurde durch Messung der Änderungen in der optischen Dichte bei 600 nm (OD₆₀₀) durch UV-Spektroskopie verfolgt.
B) Die Wachstumskurven bei 30 °C des transformierten Tryptophan-auxotrophen *E.coli*-Stamms ATCC49980 in NMM-Medium mit unterschiedlichem L-Trp- und [3,2]Sep-Konzentrationen.
   Die Zellen wurden mit zwei Plasmiden co-transformiert: Ampicillin-resistentes Plasmid pQE-30 mit dem Gen für Annexin V (PP4) und Kanamycin-resistentes Plasmid pREP4, enthaltend ein Repressorgen lacI^{q} (Qiagen). Vier verschiedene in NMM-Medium aufgezogene Kulturen wurden mit den folgenden Mengen an Tryptophan als nativem Substrat und dem Seleno-Surrogat gefüttert: **1**: 0,03 mM L-Trp; **2**: 0,015 nm L-Trp; **3**: 0,015 mM [3,2]Sep; **4**: 0,015 mM [3,2]Sep + 0,015 mM L-Trp.
   Die Mischung von L-Trp und [3,2]Sep **(4)** ermöglicht den Zellen, die stationäre Phase zu erreichen, führt aber zu einer erniedrigten Gesamtwachstumsrate. Das Wachstum wurde durch Messung der Änderungen in der optischen Dichte bei 600 nm (OD₆₀₀) durch UV-Spektroskopie verfolgt.

### Abbildung 4

I) Fluoreszenz-Emissionsspektren der nativen Proteine Annexin V (A) und Barstar b¹* (B) bzw. von deren Mutanten, angeregt bei 280 nm (blau: native Proteine; gelb: [2,3]Tpa-Proteine; rot: [3,2]Tpa-Proteine). Das Emissionsprofil von Annexin V zeigt nur den Beitrag von Tyrosin (λₘₐₓ = 307 nm) zum Gesamtprotein-Fluoreszenzprofil, während im Fall von b* die Fluoreszenz des substituierten Proteins, verglichen mit der Wildtypform, beinahe vollständig ausgelöscht ist.
II) Fluoreszenz-Emissionsspektren der nativen Proteine Annexin V (A) und Barstar b²* (B) bzw. von deren Mutanten, angeregt bei 280 nm (blau: native Proteine; rot: [3,2]Sep-Proteine). Das Emissionsprofil von [3,2]Sep-Annexin V zeigt nur den Beitrag von Tyrosin (λₘₐₓ=307 nm) zu dem Gesamtprotein-Fluoreszenzprofil, während im Fall von [3,2]Sep-b²* die Fluoreszenz des substituierten Proteins, verglichen mit der Wildtypform beinahe vollständig ausgelöscht ist.

### Abbildung 5:

I) UV-Spektren der freien Aminosäuren Tryptophan und seiner Thia-Surrogate (**A**) und der nativen Proteine und entsprechenden Mutanten (**B**: b¹*, **C**: Annexin V) bei 20°C (blau: Tryptophan und Nativproteine; gelb: [2,3]Tpa und [2,3]Tpa-Proteine; rot: [3,2]Tpa und [3,2]Tpa-Proteine).
   Bei Annexin V ist die gesamte spektrale Form und Intensität aufgrund der Tyrosindominanz in der Annexin V-Struktur (13 Tyrosine und nur 1 Tryptophan) nicht wesentlich beeinflusst. Die nativen und substituierten Annexine haben ein Absorptionsmaximum bei 277 nm.
   Dagegen verursachen die Substitutionen bei b¹* mit nur 3 Tyrosinen und 2 Tryptophan-Resten eine deutliche Blauverschiebung des Absorptionsmaximums und Veränderungen in der gesamten spektralen Form und Intensität. Die molaren Extinktionen werden für den gesamten Absorptionsbereich berechnet und in M⁻¹ cm⁻¹ angegeben, während die Absorptionsmaximum-Werte mit den entsprechenden εₘ-Werten in Tabelle 2 angegeben sind.
II) UV-Spektren der nativen Proteinen Annexin V (**A**) und b²* (**B**) bzw. von deren Mutanten bei 20 °C (blau: native Proteine; rot: [3,2]Sep-Proteine).
   Bei Annexin V ist die gesamte spektrale Form und das Absorptionsmaximum (λₘₐₓ=277 nm) von [3,2]Sep-Annexin V aufgrund der Tyrosin-Dominanz in der Annexin V-Struktur (13 Tyrosine und nur ein Tryptophan) nicht wesentlich beeinflusst, jedoch ist die Intensität des Absorptionsprofils von [3,2]Sep-Annexin V verglichen mit dem Wildtyp-Protein deutlich erhöht (ε_{M}=28400 M⁻¹cm⁻¹ zu ε_{M}=22500 M⁻¹cm⁻¹).
   Dagegen führen Substitutionen in b²* mit nur drei Tyrosinen und drei Tryptophanen zu deutlichen Änderungen in der Position des Absorptionsmaximums (blaue Verschiebung für etwa 10 nm, λ=270 nm) und insbesondere in der Absorptionsintensität. Die native Barstar-Mutante b²* hat einen Extinktionskoeffizient ε_{M} von 20460 M⁻¹cm⁻¹ bei 280 nm, während für bei [3,2]Sep-b²* ε_{M}=41580 M⁻¹cm⁻¹ bei λₘₐₓ=271 nm ist. Die molaren Extinktionen werden für den gesamten Absorptionsbereich berechnet und in dm³M⁻¹cm⁻¹ angegeben.
III) Ferne UV-CD-Profile der nativen Proteine b¹* **(A)** und Annexin V (Insert von **A**) bzw. von deren Mutanten bei 20°C. Die mittlere residuale Elliptizität ([θ]_{R}) wird in deg cm² dmol⁻¹ angegeben. Nahe UV-CD-Spektren für native Proteine bzw. deren Mutanten bei 20°C: (**B**) Annexin V, (**C**) b¹*. Die mittlere molare Elliptizität (([θ]_{M}) wird in deg cm² dmol⁻¹ angegeben (blau: Wildtypproteinformen; gelb: [3,2]Tpa-Mutanten; rot: [2,3]Tpa-Protein-Mutanten).
   Die Unterschiede in der spektralen Form im fernen UV-CD-Spektrum von [3,2]Tpa-b¹* ist möglicherweise nicht vollständig mit Änderungen in der Sekundärstruktur korreliert. Insbesondere können starke Änderungen im nahen UV-CD dieser Mutante die Form und Intensitäten von fernen UV-CD-Spektren ebenso beeinflussen.

### Abbildung 6:

Oberes Bild: Teil der Elektronendichtekarte von b²*, der die Reste W53 (rechts), W44 (oben) und W38 (links) einschließt. Die Elektronendifferenzkarte (Fₒ-F_{c}) zwischen Trp und [3,2]Sep, konturiert bei 3,5 σ (rot) ist mit der 2Fₒ-F_{c}-Elektronendichte, konturiert bei 1 σ (blau), überlagert.

Unteres Bild:
A) Elektronendichtedifferenzkarte (Fₒ-F_{c}) des Rests W187 von Annexin V, konturiert bei 3,0 σ, überlagert mit der 2Fₒ-F_{c}-Elektronendichtekarte (1,3 σ).
B) Elektronendichtekarte (2Fₒ-F_{c}) bei 1,3 σ mit der [3,2]Sep-Seitenketten-Modellstruktur an Position 187 in Annexin V.

## Patentansprüche

1. Protein, Peptid oder peptidische Leitstruktur,
**dadurch gekennzeichnet, dass** es mindestens ein Chalkogen-enthaltendes Aminosäureanalogon von Tryptophan und/oder mindestens ein Halogen-enthaltendes Aminosäureanalogon von Tryptophan umfasst, wobei das Chalkogen aus der Gruppe, bestehend aus Schwefel, Selen und Tellur, ausgewählt ist, und das Halogen aus der Gruppe, bestehend aus Brom und Iod, ausgewählt ist, und Peptide, die Brom-enthaltende Aminosäureanaloga von Tryptophan umfassen, ausgeschlossen sind.

2. Protein, Peptid oder peptidische Leitstruktur nach Anspruch 1,
**dadurch gekennzeichnet, dass** die Aromatizität in dem dem Indolring von Tryptophan entsprechenden Ringsystem des Aminosäureanalogons erhalten ist.

3. Protein, Peptid oder peptidische Leitstruktur nach Anspruch 1 oder 2,
**dadurch gekennzeichnet, dass** das dem Indolring von Tryptophan entsprechende Ringsystem zwei kondensierte Fünfringe umfasst, wobei sich eine NH-Gruppe in dem einen Fünfring befindet und ein Chalkogen in dem anderen Fünfring eingebaut ist.

4. Protein, Peptid oder peptidische Leitstruktur nach einem der vorangehenden Ansprüche,
**dadurch gekennzeichnet, dass** das Chalkogen Selen ist.

5. Protein, Peptid oder peptidische Leitstruktur nach einem der vorangehenden Ansprüche,
**dadurch gekennzeichnet, dass** das Aminosäureanalogon von Tryptophan aus der Gruppe, bestehend aus β-Selenolo[3,2-b]pyrrolyl-L-alanin und β-Selenolo[2,3-b]pyrrolyl-L-alanin, ausgewählt ist.

6. Protein, Peptid oder peptidische Leitstruktur nach einem der vorangehenden Ansprüche,
**dadurch gekennzeichnet, dass** das dem Indolring von Tryptophan entsprechende Ringsystem mit Halogenen substituiert ist.

7. Protein, Peptid oder peptidische Leitstruktur nach einem der vorangehenden Ansprüche,
**dadurch gekennzeichnet, dass** das Aminosäureanalogon von Tryptophan aus der Gruppe, bestehend aus β-Thieno[3,2-b]pyrrolyl-L-alanin, β-Thieno[2,3-b]pyrrolyl-L-alanin, β-Tellurolo[3,2-b]pyrrolyl-L-alanin und β-Tellurolo[2,3-b]pyrrolyl-L-alanin, 4-Bromo-Tryptophan, 5-Bromo-Tryptophan, 6-Bromo-Tryptophan, 4-Jodo-Tryptophan, 5-Jodo-Tryptophan und 6-Jodo-Tryptophan ausgewählt ist.

8. Protein, Peptid oder peptidische Leitstruktur nach einem der vorangehenden Ansprüche,
**dadurch gekennzeichnet, dass** das Aminosäureanalogon von Tryptophan eine kanonische Aminosäure eines nativen Proteins, eines nativen Peptids oder einer nativen peptidischen Leitstruktur ersetzt.

9. Protein, Peptid oder peptidische Leitstruktur nach einem der vorangehenden Ansprüche,
**dadurch gekennzeichnet, dass** alle Tryptophanreste der Sequenz eines nativen Proteins, eines nativen Peptids oder einer nativen peptidischen Leitstruktur durch Aminosäureanaloga von Tryptophan ersetzt sind.

10. Verfahren zur Markierung von Proteinen, Peptiden oder peptidischen Leitstrukturen für kristallographische und/oder spektroskopische Untersuchungen,
**dadurch gekennzeichnet, dass** mindestens ein Chalkogen-enthaltendes Aminosäureanalogon von Tryptophan und/oder mindestens ein Halogen-enthaltendes Aminosäureanalogon von Tryptophan in ein Protein, ein Peptid oder eine peptidische Leitstruktur eingebaut wird, wobei das Chalkogen aus der Gruppe, bestehend aus Schwefel, Selen und Tellur, ausgewählt wird, und das Halogen aus der Gruppe, bestehend aus Brom und Iod, ausgewählt wird, und Peptide, die Brom-enthaltende Aminosäureanaloga von Trypthophan umfassen, ausgeschlossen sind.

11. Verfahren nach Anspruch 10,
**dadurch gekennzeichnet, dass** der Einbau des Aminosäureanalogons von Tryptophan in das Protein *in vivo* in einer Wirtszelle erfolgt.

12. Verfahren nach Anspruch 11,
**dadurch gekennzeichnet, dass** der Einbau des Aminosäureanalogons von Tryptophan in das Protein die folgenden Schritte umfasst:
a) Bereitstellen einer Wirtszelle mit einer für das Protein kodierenden Nukleinsäuresequenz in operativer Verknüpfung mit einer regulierbaren Expressionskontrollsequenz,
b) Kultivieren der Zelle unter Bedingungen, bei denen die Transkription der für das Peptid oder Protein kodierenden Nukleinsäuresequenz weitgehend abgeschaltet ist,
c) Zugeben des Aminosäureanalogons von Tryptophan, und
d) Induzieren der Expression des Peptids oder Proteins.

13. Verfahren nach Anspruch 12,
**dadurch gekennzeichnet, dass** eine Wirtszelle verwendet wird, die für eine durch das Aminosäureanalogon von Tryptophan zu ersetzende kanonische Aminosäure auxotroph ist, und bei Schritt a) die zu ersetzende kanonische Aminosäure dem Medium zugesetzt wird.

14. Verfahren zur Bestimmung der dreidimensionalen Struktur von Proteinen, Peptiden oder peptidischen Leitstrukturen mittels Kristallstrukturanalyse, umfassend die folgenden Schritte:
a) Herstellung eines Proteins, eines Peptids oder einer peptidischen Leitstruktur, welches bzw. welche mindestens ein Chalkogen-enthaltendes Aminosäureanalogon und/oder mindestens ein Halogen-enthaltendes Aminosäureanalogon von Tryptophan umfasst, wobei das Chalkogen aus der Gruppe, bestehend aus Schwefel, Selen und Tellur, ausgewählt wird, und das Halogen aus der Gruppe, bestehend aus Brom und Iod, ausgewählt wird, durch Substitution mindestens eines Tryptophanrests des nativen Proteins, des nativen Peptids oder der nativen peptidischen Leitstruktur durch ein Aminosäureanalogon von Tryptophan;
b) Kristallisierung des in Schritt a) hergestellten Proteins, Peptids bzw. der peptidischen Leitstruktur;
c) Kristallstrukturanalyse des Kristalls des Proteins, Peptids bzw. der peptidischen Leitstruktur aus Schritt b).

15. Verfahren nach Anspruch 14,
**dadurch gekennzeichnet, dass** zur Bestimmung der Phasen der Streuwellen (Strukturfaktoren) in Schritt c) nach der MAD-Methode das Aminosäureanalogon von Tryptophan ausgewählt wird aus der Gruppe, bestehend aus Selen-, Brom- und Iod-enthaltenden Aminosäureanaloga.

16. Verfahren nach Anspruch 15,
**dadurch gekennzeichnet, dass** das Aminosäureanalogon von Tryptophan aus der Gruppe, bestehend aus β-Selenolo[3,2-b]pyrrolyl-L-alanin, β-Selenolo[2,3-b]pyrrolyl-L-alanin, 4-Bromo-Tryptophan, 5-Bromo-Tryptophan, 6-Bromo-Tryptophan, 4-Jodo-Tryptophan, 5-Jodo-Tryptophan und 6-Jodo-Tryptophan, ausgewählt wird.

17. Verfahren nach Anspruch 14,
**dadurch gekennzeichnet, dass** zur Bestimmung der Phasen der Streuwellen (Strukturfaktoren) in Schritt c) nach der Methode der multiplen Schweratomderivate das Aminosäureanalogon von Tryptophan ausgewählt wird aus der Gruppe, bestehend aus Schwefel-, Tellur-, Brom- und Iod-enthaltenden Aminosäureanaloga.

18. Verfahren nach Anspruch 17,
**dadurch gekennzeichnet, dass** das Aminosäureanalogon von Tryptophan ausgewählt wird aus der Gruppe, bestehend aus β-Thieno[3,2-b]pyrrolyl-L-alanin, β-Thieno[2,3-b]pyrrolyl-Lalanin, β-Tellurolo[3,2-b]pyrrolyl)-L-alanin, β-Tellurolo[2,3-b]pyrrolyl)-L-alanin, 4-Bromo-Tryptophan, 5-Bromo-Tryptophan, 6-Bromo-Tryptophan, 4-Jodo-Tryptophan, 5-Jodo-Tryptophan und 6-Jodo-Tryptophan.

19. Verwendung von Chalkogen- und/oder Halogen-enthaltenden Aminosäureanaloga von Tryptophan zur Markierung von Proteinen, Peptiden oder peptidischen Leitstrukturen für Fluoreszenz-spektroskopische Untersuchungen.

20. Verwendung von Chalkogen-enthaltenden Aminosäureanaloga von Tryptophan zur Markierung von Proteinen, Peptiden oder peptidischen Leitstrukturen für UVspektroskopische Untersuchungen.

21. Verwendung von Chalkogen- und/oder Halogen-enthaltenden Aminosäureanaloga, insbesondere von ⁷⁷Se-Analoga, von Tryptophan zur Markierung von Proteinen, Peptiden oder peptidischen Leitstrukturen für NMR-spektroskopische Untersuchungen.

22. Pharmazeutische Zusammensetzung,
**dadurch gekennzeichnet, dass** sie als Wirkstoff mindestens ein Chalkogen-enthaltendes Aminosäureanalogon von Tryptophan eingebaut in einen Träger, ausgewählt aus einem Protein, einem Peptid oder einer peptidische Leitstruktur, umfasst.

23. Pharmazeutische Zusammensetzung nach Anspruch 22,
**dadurch gekennzeichnet, dass** das Aminosäureanalogon von Tryptophan β-Thieno[3,2-b]pyrrolyl-L-alanin oder β-Thieno[2,3-b]pyrrolyl-L-alanin ist.
